(19) Europäisches Patentamt
European Patent Office
Office européen des brevets

(11) **EP 2 297 355 B1**

(12) **EUROPEAN PATENT SPECIFICATION**

(45) Date of publication and mention
of the grant of the patent:
**02.10.2013 Bulletin 2013/40**

(51) Int Cl.:
***C12Q 1/68*** *(2006.01)*

(21) Application number: **09766129.2**

(22) Date of filing: **18.06.2009**

(86) International application number:
**PCT/GB2009/001536**

(87) International publication number:
**WO 2009/153568 (23.12.2009 Gazette 2009/52)**

(54) **METHOD FOR DETERMINING DNA COPY NUMBER BY COMPETITIVE PCR**

VERFAHREN ZUR BESTIMMUNG DER DNA-KOPIENZAHL MITTELS KOMPETITIVER PCR

MÉTHODE POUR DÉTERMINER UN NOMBRE DE COPIES D'ADN PAR PCR COMPÉTITIVE

(84) Designated Contracting States:
**AT BE BG CH CY CZ DE DK EE ES FI FR GB GR
HR HU IE IS IT LI LT LU LV MC MK MT NL NO PL
PT RO SE SI SK TR**

(30) Priority: **20.06.2008 GB 0811500**

(43) Date of publication of application:
**23.03.2011 Bulletin 2011/12**

(73) Proprietor: **University College Cardiff
Consultants Limited
Cardiff CF24 0DE (GB)**

(72) Inventors:
• **WILLIAMS, Nigel Melville
Cardiff CF 14 4XN (GB)**
• **O'DONOVAN, Michael Conlon
Cardiff CF14 4XN (GB)**
• **OWEN, Michael John
Cardiff CF144XN (IN)**

(74) Representative: **Fyles, Julie Marie
Symbiosis IP Limited
Basepoint Business Centre
Vale Business Park
Crab Apple Way
Evesham
Worcestershire WR11 1GP (GB)**

(56) References cited:
**WO-A-2004/022721      WO-A-2006/011738
US-A1- 2005 059 041**

• **DEUTSCH S ET AL: "Detection of aneuploidies
by paralogous sequence quantification"
JOURNAL OF MEDICAL GENETICS, BMJ
PUBLISHING GROUP, LONDON, GB, vol. 41, no.
12, 1 December 2004 (2004-12-01), pages 908-915,
XP009096666 ISSN: 0022-2593**
• **GOIDTS V ET AL: "O15: Identification of large-
scale human-specific copy number variations by
array comparative genomic hybridization"
EUROPEAN JOURNAL OF MEDICAL GENETICS,
ELSEVIER, NL, vol. 48, no. 4, 1 October 2005
(2005-10-01), pages 478-479, XP025341865 ISSN:
1769-7212 [retrieved on 2005-10-01]**
• **ARMOUR JOHN A L ET AL: "Accurate, high-
throughput typing of copy number variation
using paralogue ratios from dispersed repeats"
NUCLEIC ACIDS RESEARCH, OXFORD
UNIVERSITY PRESS, SURREY, GB, vol. 35, no. 3,
14 December 2006 (2006-12-14), page E19,
XP002473374 ISSN: 0305-1048 cited in the
application**
• **WILLIAMS NIGEL M ET AL: "Analysis of copy
number variation using quantitative interspecies
competitive PCR." NUCLEIC ACIDS RESEARCH
OCT 2008, vol. 36, no. 17, October 2008 (2008-10),
page e112, XP002539705 ISSN: 1362-4962**

**Description**

The present invention relate to a method for accurately determining the number of copies of DNA (copy number) carried at a specific locus.

It is well recognised that rare large microscopic genomic abnormalities are associated with disease (Chance et al. 1993; Driscoll et al. 1992). Over recent years a number of landmark studies have shown that smaller submicroscopic DNA copy-number variants (CNVs) (typically segments >1kb that are deleted, duplicated or inserted) are an important source of variation in the human genome (reviewed in (Feuk et al. 2006)). That CNVs are a common source of genetic variation in healthy individuals (Conrad et al. 2006; Hinds et al. 2006; McCarroll et al. 2006; Redon et al. 2006) implies that some result in no obvious phenotypic changes. However as CNVs can disrupt entire genes and regulatory regions (Hinds et al. 2006; McCarroll et al. 2006; Sebat et al. 2004; Sharp et al. 2005) an increasing number have been shown to make an important contribution to human phenotypic diversity and play a role in disease susceptibility (Aitman et al. 2006; Gonzalez et al. 2005; Lucito et al. 2007; Sebat et al. 2007). Consequently, the identification of disease related CNVs is important both clinically and for studies aiming to identify disease related aetiological pathways. A key step in the investigation of CNVs will involve the analysis of specific loci in disease association studies and to achieve this CNVs will have to be accurately typed in large clinical cohorts.

Array based methodologies (Carter 2007) have allowed large numbers of CNVs to be detected and characterised. Such methods have however been developed mainly for studies aimed at accurately detecting CNVs and, in order to maintain a low false positive detection rate, typically set stringent inclusion criteria which can often result in an inflated false negative rate (McCarroll and Altshuler 2007). It is therefore generally accepted that a confirmatory analysis using an independent method is required to accurately determine the frequency of CNVs identified by array based methods.

Association studies require an extremely accurate determination of the genotype at each locus for large numbers of samples. To perform association analysis on CNVs it is essential that all levels of copy number are accurately determined in each sample (0, 1, 2, 3, etc.). Estimations of DNA copy number from data generated by array based studies often does not fall into discrete categories and instead can typically form a continuous distribution. In fact, a recent assessment of 1500 CNVs identified by array based discovery studies revealed that the call data (i.e. Accurate determination of DNA copy number) of only 70 were of the standard expected of genotyping assays (McCarroll and Altshuler 2007). This problem is likely to be an even greater issue for smaller CNVs which contain lower numbers of probes. It is well recognised that even relatively small error rates can dramatically reduce the power of an association study (Moskvina et al. 2006), therefore inaccurate estimates of copy number are unacceptable and influence both type I (i.e. false positive) and type II (i.e. false negative) error. To address this, some have attempted to fit such data into the appropriate discrete categories (Redon et al. 2006; Stranger et al. 2007), however such approaches have major limitations (McCarroll 2008) and are likely to be more amenable to some CNVs than others. Given this, a more satisfactory approach would be to develop assays that estimate DNA copy number with sufficiently high quality that artefacts introduced by experimental noise are suitably reduced. Such approaches would also facilitate the economic association analysis of CNVs in large replication samples. As a result of these issues, the development of methods that allow efficient, accurate and affordable measurement of genomic copy number polymorphisms in clinical cohorts has been recently recognised as one of the most pressing needs in CNV research (McCarroll and Altshuler 2007; Todd 2006).

A number of methods exist that allow accurate quantitation of DNA copy number (Armour et al. 2007; Armour et al. 2000; Charbonnier et al. 2000; Heid et al. 1996; Schouten et al. 2002; Suls et al. 2006) however none are particularly well suited to large scale association analysis, either because the assay requires intensive optimisation or because the analysis platform on which they have been developed is not best applied to high throughput studies. Competitive PCR is an established technique which involves amplifying a test DNA sequence in the presence of a competitor sequence which is identical apart from a single nucleotide which allows both sequences to be distinguished. Given a known concentration of the competitor, the test sequence can be accurately quantified. Topically, the source of the competitor is a synthetic DNA sequence, however the requirement for a competitor to be synthesised to match each test locus is often impractical for studies analysing large numbers of loci. To avoid this, others have shown that very accurate estimates of DNA copy number can be obtained by amplifying the test locus in the presence of a paralogous locus of known copy number (Armour et al. 2007). However, not all test loci will have a non-deleted/duplicated paralogous sequence, limiting the number of assays that can be analysed by this approach.

The present inventors have adopted an alternative approach and applied competitive PCR to determine DNA copy number by exploiting the high degree of conservation between the orthologous genomic sequence of two closely related species. As a proof of concept the authors have achieved this by using the entire genome of a single chimpanzee (*Pan troglodytes*) as a competitor to accurately determine the copy number at specific loci in the human (*Homo sapiens*) genome.

Therefore, in a first aspect of the invention, there is provided a method for determining copy number at a specific test locus in DNA of an exploratory species, the method comprising the steps of:

(i) co-amplifying a DNA sample of the exploratory species (the exploratory sample) comprising the test locus with at least one competitor sample comprising an orthologous DNA sequence derived from a closely related species;

(ii) targeting nucleotides within the orthologous DNA sequence that are not conserved between the two species using at least one appropriate molecular assay in order to distinguish between the DNA sequences of the exploratory and competitor samples;

(iii) quantifying the amount of the DNA present in the exploratory species for each assay targeting a non-conserved nucleotide by comparing the amount of the DNA present in the exploratory species with that in the competitor sample; and

(iv) determining a quantitative estimate of the DNA copy number at the test locus in the exploratory species;

wherein the competitor sample comprises DNA which is orthologous to the DNA sequence of the exploratory species and contains at least one non-conserved nucleotide such that the exploratory and competitor samples can be distinguished by at least one appropriate molecular assay.

The amplification step may be carried out by any suitable method, for example polymerase chain reaction (PCR).

In one example of the invention, the exploratory species may be *Homo sapiens.* This is particularly useful when the method is used in disease diagnosis or association studies or in genetic fingerprinting methods as discussed below. When the exploratory species is *Homo sapiens,* the closely related species may be an ape species, for example chimpanzee (*Pan troglodytes)* and this is the example used by the inventors to demonstrate proof of concept. However, when the exploratory species is *Homo sapiens*, other closely related species, particularly apes such as a gorilla species or bonobo (*Pan paniscus*) may also be used.

The concept of a closely related species is well understood by those skilled in the art of genetics and such a person would have no difficulty in selecting a suitable closely related species for a selected exploratory species since it is known that closely related species are selected on the basis that the specific sequence (s), to which the primers bind, should have sufficient homology between the two species selected to enable the simultaneous analysis of both the target and the competitor loci.

The exploratory sample may comprise more than one test locus and, indeed may comprise multiple test loci, for example up to 5, up to 10, up to 20 up to 30, up to 40 or up to 50 test loci. In some cases, the number of test loci may be significantly greater than this, for example several hundred, several thousand or even greater than a million. All of the test loci may be analysed simultaneously using the method of the invention.

The exploratory sample may comprise DNA from, or consisting of, one or more chromosome or even the entire genome of the individual of the exploratory species.

The test locus or loci may have been predetermined as areas of interest before the method is carried out. Alternatively, a test locus may be previously unknown and may simply be a region which, after the steps of the method have been carried out, is demonstrated to have a DNA copy number gain or loss relative to the reference or competitor genome.

The method of the invention may thus be used to analyse large numbers of copy number polymorphisms in a sample and thereby obtain a unique genetic profile of an individual. It is therefore potentially useful in "DNA fingerprinting" methods and in building genetic data bases. In such methods, the test loci are preferably predetermined but it is possible that undetermined test loci are used so that the method is used to detect all copy number variations of an individual within the exploratory sample.

The competitor sample may comprise DNA from, or consisting of, one or more chromosome or even the entire genome of a single individual of the competitor species and this latter option is advantageous because it results in the requirement for only a single competitor sample for all assays and thus dramatically increases the potential for large numbers of loci to be analysed in multiplex. It is also useful in cases when the test loci have not been predetermined before the method has been carried out.

The test locus may be or include a DNA sequence for which copy number variation (either copy gain or copy loss) is associated with phenotypic variation in the exploratory species, for example Parkinson's disease, DiGeorge's syndrome (22q11 DS), cancer, Charcot-Marie Tooth, CYP450 metabolism.

As a control, the copy number at a test locus may be compared with the copy number at an internal control locus. Internal control loci may be DNA sequences for which there is no evidence for copy gain or copy loss in the test or exploratory species.

The non-conserved nucleotides may be designed by aligning the respective orthologous genomic sequences of the exploratory and competitor species and identifying nucleotides which differ between the two species. Molecular assays can then be designed to target the non-conserved nucleotides.

For each assay targeting a non-conserved nucleotide, the amount of the allele of the exploratory species present (*exploratory*DNA$^{relative}$) relative to that of the competitor species may be determined using any appropriate method, laboratory instrumentation and software. Examples of laboratory instrumentation and software which may be used include, for example the Sequenom MassARRAY™ system. With reference to the respective standard curve, the quantity of DNA present at each assay locus (*exploratory*DNA$^{quant}$) in the genome of the exploratory species sample may then be estimated using an equation such as

$$exploratoryt\text{DNA}^{quant} = \text{EXP}[(exploratory\text{DNA}^{relative} \times m) + c]$$

The estimated DNA copy number at a given test locus in the genome of the exploratory species sample may be determined by any appropriate method, for example as the ratio of *exploratory*DNA$^{quant}$ determined at the test and internal control loci respectively: a ratio of 1:1 being expected in the absence of a CNV, 0.5 for a heterozygous deletion, 1.5 for a heterozygous duplication. In this case, dividing each ratio by 0.5 would provide an estimate of the DNA copy number at the test locus.

In some cases, as discussed below, it is advantageous to use multiple assays targeting different non-conserved nucleotides from the same locus.

These can be used to determine the average DNA$^{quant}$ for the exploratory species at the test and internal control loci respectively and thus more accurately determine the average copy number at the test locus.

In some cases, as discussed below, it is advantageous to use multiple references, i.e. exploratory or internal control, loci or locus. This improves the accuracy of the assay.

Since the method of the invention enables the copy number of a DNA sequence to be determined, it is also of use in diagnosing diseases or phenotypes associated with genomic copy number variation.

Therefore, in a second aspect of the invention there is provided a method for diagnosing a disease or specific phenotype know to be associated with a change in DNA copy number of a specific chromosomal DNA sequence (the test locus) in the genome of an exploratory species, the method comprising:

(i) co-amplifying a sample of DNA of the exploratory species comprising the test locus (the exploratory sample) with at least one competitor sample comprising an orthologous DNA sequence derived from a closely related species;
(ii) targeting nucleotides within the orthologous DNA sequence that are not conserved between the two species using at least one appropriate molecular assay in order to distinguish between the DNA sequences of the exploratory and competitor samples;
(iii) quantifying the amount of the DNA present in the exploratory species for each assay targeting a non-conserved nucleotide by comparing the amount of the DNA present in the exploratory species with that in the competitor sample; and
(iv) determining a quantitative estimate of the DNA copy number at the test locus in the exploratory species;

wherein the competitor sample comprises DNA which is orthologous to the DNA sequence of the exploratory sample and contains at least one non-conserved nucleotide such that the exploratory and competitor samples can be distinguished by at least one appropriate molecular assay and wherein either a gain or loss of normal DNA copy number at the test locus is associated with the disease or phenotype.

The test locus may be a DNA sequence for which copy number variation (either copy gain or copy loss) is associated with phenotypic variation in the exploratory species, for example Parkinson's disease, DiGeorge's syndrome (22q11 DS), cancer, Charcot-Marie Tooth, CYP450 metabolism.

Other features are as for the first aspect of the invention.

The method of the invention may alternatively be used in determining an association between a copy number variant at a locus or loci in genomic DNA and a disease or condition.

Therefore, in a third aspect of the invention, there is provided a method of determining an association between a copy number variant (CNV) in genomic DNA and a disease or condition, the method comprising:

(i) co-amplifying a sample of DNA from a patient of the exploratory species with the disease or condition (the exploratory sample) with at least one competitor sample comprising an orthologous DNA sequence derived from a closely related species;
(ii) targeting nucleotides within the orthologous DNA sequence that are not conserved between the two species using at least one appropriate molecular assay in order to distinguish between the DNA sequences of the exploratory and competitor samples;
(iii) quantifying the amount of the DNA present in the exploratory species for each assay targeting a non-conserved nucleotide by comparing the amount of the DNA present in the exploratory species with that in the competitor sample; and
(iv) determining a quantitative estimate of the DNA copy number at one or more test loci in the exploratory species, wherein a copy number variant at a test locus may indicate association with the disease or condition;

wherein the DNA sequence of the competitor species is orthologous to the DNA sequence of the exploratory species and contains at least one non-conserved nucleotide such that the exploratory and competitor sequences can be distinguished by at least one appropriate molecular assay.

In this aspect of the invention, the test loci may not be known at the beginning of the process. Alternatively, the test locus or loci may be selected to be in an area of the genome which is suspected of being associated with the disease or condition. The exploratory sample may comprise a part of the genome including the test locus or loci or the entire genome of the patient.

It is greatly preferred that the method is repeated using exploratory samples derived from multiple patients in order to obtain a valid association between copy number variation at particular test loci and the disease or condition.

It is also preferred that in order to validate the results, steps (i) to (iv) of the method are also carried out using reference samples from one or more patients of the exploratory species who do not suffer from the disease or condition. The reference samples will be chosen such that they are analogous to the exploratory samples, and therefore will be of the same length and from the same area of the genome as the exploratory samples.

The more exploratory and reference samples used in the method, the greater the accuracy of any association between a copy number variation at a test locus and a disease or condition.

Other preferred features are as described for the first aspect of the invention.

The invention will now be described in greater detail with reference to the Examples and to the drawings in which the authors have as a proof of concept, accurately determined the copy number at specific loci in the human (Homo sapiens) genome by using the entire genome of a single chimpanzee (*Pan troglodytes*) as a competitor.

**Table** 1: **Copy number estimates at the PARK2 locus.**
Copy number at PARK2 exons was determined by MLPA and qicPCR in 2 patients containing previously characterised mutations (highlighted in grey) and 10 healthy controls. Mean, standard deviation and coefficient of variance were calculated for each qicPCR assay in the 10 control samples only.

**Figure 1: Schematic representation of the principle of quantitative interspecies competitive PCR (qicPCR).**
Human and pan troglodytes specific alleles are represented by alleles 'C' and 'G' respectively.

**Figure 2: Comparison of the copy number at the PARK2 locus as determined by MPLA and qicPCR.**
Copy number estimates of PARK2 exons 3, 4, 5, 6, 8, 9, 10 and 11 as determined by qicPCR and MLPA in 2 patients with previously characterised PARK2 mutations. The assays of exons 3 (a and b) and exon 6 which detect the deletion and duplication in our test samples PD-01 and PD-02 respectively are highlighted.

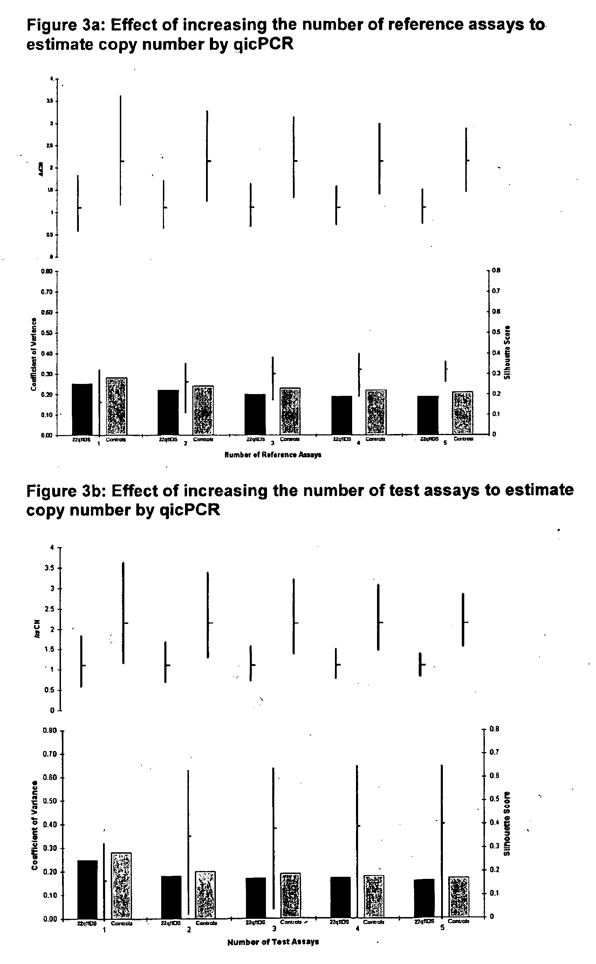

**Figure 3a: Effect of increasing the number of reference samples to estimate copy number by qicPCR**

**Figure 3b: Effect of increasing the number of test assays to estimate copy number by qicPCR**
Upper vertical bars represent the range of hsCN determined by qicPCR for 22q11DS patients and controls while histograms represent the CV. The ranges of silhouette scores determined at each parameter are displayed as lower vertical bars. For each measure the mean is indicated as a small horizontal bar.

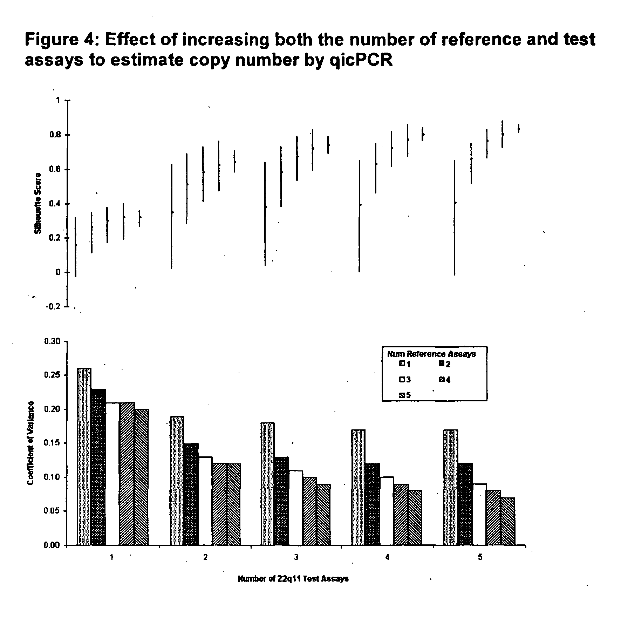

**Figure 4: Effect of increasing both the number of reference and test assays to estimate copy number by qicPCR**
hsCN was determined at 22q11 for all combinations of 1 to 5 test and reference assays in 10 patients with 22q11DS and 10 non-deleted controls. For simplicity the CV of only the 22q11 DS samples is presented however an analogous pattern was seen in the data for the non-deleted controls.

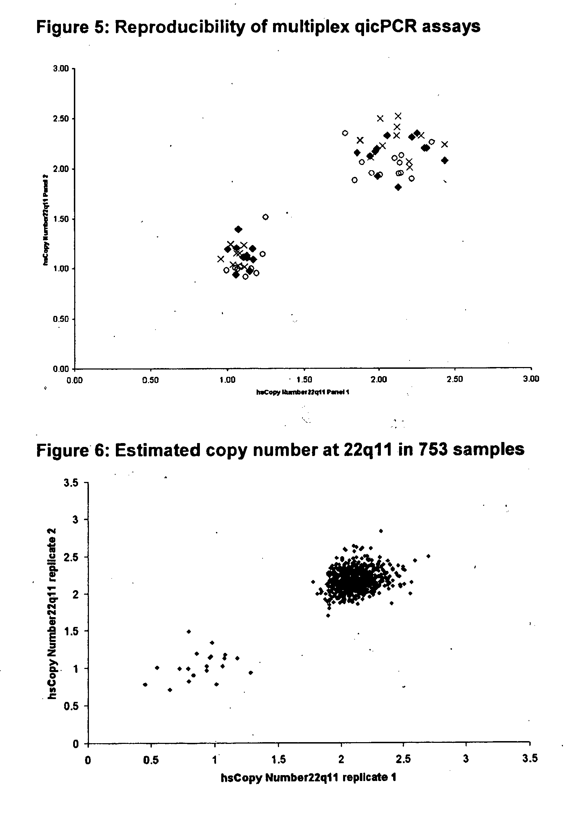

**Figure 5: Reproducibility of multiplex qicPCR assays**
hsCN was determined at 22q11 using two independent multiplex qicPCR panels in ten 22q11 DS patients (left-hand cluster) and 10 non-deleted controls (right-hand cluster). Each panel was analysed in triplicate with replicates 1, 2 and 3 for each qicPCR panel being represented as triangles, crosses and circles respectively.

**Figure 6: Estimated copy number at 22q11 in 753 samples**
Replicate experiments of a single multiplex qicPCR panel to determine hsCN at 22q11 in twenty 22q11DS patients (left-hand cluster) and 733 controls (right-hand cluster).

## EXAMPLE

## Methods

## Samples

All subjects were unrelated and provided written informed consent to participate in genetic studies.

Parkinsons disease (PD) samples: Our initial analysis was based on two patients diagnosed with PD who had been previously characterised as carrying PARK2 mutations; patient PD-01 was hemizygously deleted at exon 3 while patient PD-02 carried a heterozygous duplication of exon 6. All mutations were molecularly confirmed by MLPA.

22q11DS samples: We analysed 20 unrelated individuals who carried hemizygous deletions at 22q11 as determined by fluorescence in situ hybridisation using the N25 probe (Oncor Inc, Gaithersburg, Md).

Control samples: All 733 unrelated individuals had been collected for use as controls, details of which have been previously described (Williams et al. 2004). The 10 samples used as controls against the PD patients had previously not been screened for the PARK2 mutations, however given the rarity of such pathogenic mutations it is unlikely that they were carriers. All samples had however been previously excluded for the presence of typical 22q11 deletions region (Ivanov et al. 2003).

Chimpanzee: Pan Troglodytes DNA was obtained from the cell line EB176 (JC) deposited at ECACC (http: //www.ecacc.org.uk/).

**Molecular assays**

*MLPA assay*

Samples were analysed using the SALSA P051/P052 Parkinson MLPA probe kit (MRC Holland) following manufacturer's instruction. Each kit contains a probe for exons 1 to 12 of the PARK2 gene. All steps were performed on the same MJ thermocycler. Briefly, 100ng of DNA was denatured at 98°C and hybridised to the probes by incubation (16-17 hours, 60°C) with 1.5$\mu$l SALSA probemix and 1.5$\mu$l of MLPA buffer. The ligation reaction was carried out by incubating the 8$\mu$l of hybridised product and 32$\mu$l of Ligase-65 mix for 15 mintutes at 54°C, followed by 5 minutes at 98°C. The PCR was performed in a 50$\mu$l reaction using 10$\mu$l of ligation product, 4$\mu$l of 10x SALSA PCR buffer and 10$\mu$l of Polymerase mix. The PCR cycling conditions were 60°C hotstart, followed by 35 cycles of 95°C for 30 seconds, 60°C for 30 seconds, and 72°C for 60 seconds, followed by 72°C for 20 minutes. The PCR product was analysed on an ABI 3100 Sequencer (Applied Biosystems) with a GeneScan500 Rox internal size standard.

**qicPCR assays**

*PCR*

PCR was performed on MJ thermocyclers in a 5$\mu$l reaction using 12ng of dried-down genomic *hs*DNA, 12ng of *pt*DNA, 0.5pmol of each primer in multiplex, 250$\mu$M dNTPs, 0.325$\mu$l of 10X 20mM MgCl PCR Buffer and 0.1 units of Hotstar Taq polymerase (Qiagen). The PCR cycling parameters were 95°C for 15 minutes, followed by 45 cycles of 94°C for 20 seconds, 56°C for 30 seconds, and 72°C for 1 minute, followed by 72°C for 3 minutes.

*Primer extension*

Primer extension was performed on MJ thermocyclers in 9$\mu$l reactions using the 7$\mu$l SAP-treated PCR product, 6.6pmol to 13.3 pmol of each primer in multiplex, 0.25$\mu$l of iplex buffer, 0.25$\mu$l of iplex termination mix and 0.05$\mu$l of iplex enzyme (Sequenom). The PCR cycling parameters were 94°C for 30 seconds, followed by 40 cycles of 94°C for 5 seconds, and a nested 5 cycles of 52°C for 5 seconds and 80°C for 5 seconds, followed by 72°C for 3 minutes.

*qicPCR assay selection*

Interspecies sequence differences were identified by aligning the respective genomic DNA sequences of human (homo sapiens) and chimpanzee (Pan troglodytes) using UCSC Blat (http: // genome.ucsc.edu/) . Nucleotides that differed between human and chimp were identified and with reference to the UCSC genome browser human March 2006 build and the chimpanzee genome database at the Broad Institute (http: //www.broad.mit.edu/ftp/pub/ assemblies/ mammals/ chimp_ SNPs/), known human and chimpanzee SNPs were excluded. Single nucleotide extension assays were then designed to target the non- conserved nucleotides using Sequenom Assay Design v3.1 software.

To study the mutations in the PD patients we used the human reference sequence at the PARK2 locus (NCBI build 36.1, chr6: 161, 689, 662- 162, 784, 495) with its orthologous sequence in Pan troglodytes (genome build 2 version 1, chr6: 164, 335, 097- 165, 462, 200) . To study the deletion at 22q11 we aligned the human genome NCBI build 36.1, chr22: 18131905- 18, 222, 087 with its orthologous region in Pan troglodytes (genome build 2 version 1, chr22: 18221742-18316567) . Primer extension assays (Sequenom iplex™) were then designed to target a total of 30 nucleotides that were not conserved between the two species at the PARK2 locus (20 assays) and at 22q11 (10 assays) (Supplementary table 1) . Internal control assays were designed by identifying nucleotides that were not conserved between human and chimpanzee at a number of autosomal regions that had showed no previous evidence for harbouring common CNVs (supplementary table 2) . qicPCR assays targeting the PARK2 locus were designed as two independent multiplex reactions each containing 9 and 11 independent test assays as well as a single reference assay. qicPCR assays targeting 22q11 were designed as two independent multiplex reactions each containing five 22q11 test assays and five reference assays.

Primers used in qicPCR are shown in Supplementary Tables 1 and 2.

*Standard curve*

The genomic DNA from a single human reference sample was quantified to 32ng/ul by pico green analysis and then serially diluted to 16ng/ul, 8ng/ul, 4ng/ul, 2ng/ul and 1ng/ul. For each titrate, PCR followed by primer extension was then performed using 3ul of hsDNA and 3ul of ptDNA (4ng/ul) as per the manufacturer's instructions with the iplex chemistry. The peak areas of the extended primers specific to the respective human and chimpanzee alleles were determined following analysis on a Sequenom MassARRAY™ system. The amount of hsDNA relative to that of ptDNA at each locus (hsDNArelative) was then estimated by

$$hsDNArelative = hs\ peak\ height/(hs\ peak\ height + pt\ peak\ height)$$

A reference standard curve was generated by plotting hsDNArelative (x-axis) against the log of known hsDNA concentration (y-axis) and from this the slope and intercept was estimated from the equation of the straight line, $y = mx + c$. This simple and rapid procedure was performed for each primer extension assay.

*qicPCR analysis*

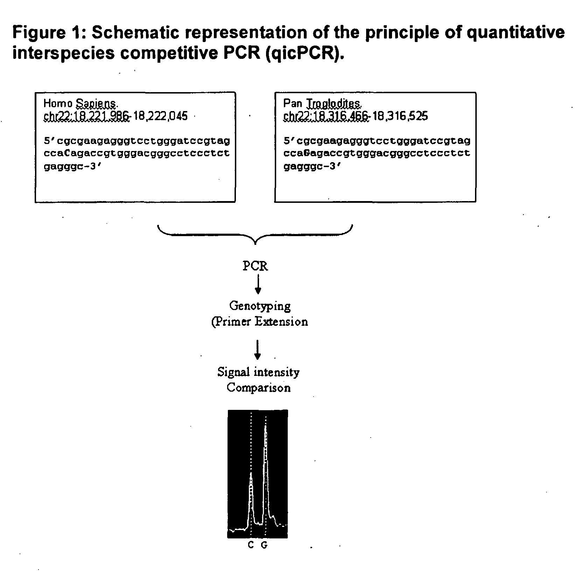

For each test sample, the assay was performed using equal concentrations of genomic hsDNA and ptDNA (3ul of each at approximately 4ng/ul) as described above. The area under peaks corresponding to extended primer peaks of the respective human and chimpanzee nucleotides (figure 1) was then determined and the data were used to estimate hsDNArelative for each primer extension assay. With reference to the respective standard curve, the quantity of hsDNA present in each primer extension assay (hsDNAquant) was then estimated by

$$hsDNAquant = EXP[(hsDNArelative \times m) + c]$$

where m and c were derived from the reference standard curve.

The estimated hsDNA copy number at the test locus was determined as the ratio of hsDNAquant determined at the test and internal control loci respectively: a ratio of 1:1 being expected in the absence of a CNV, 0.5 for a heterozygous deletion, 1.5 for a heterozygous duplication. Dividing each ratio by 0.5 provided an estimate of the human DNA copy number at the test locus (hsCN). When multiple test assays from the same locus were used we first determined the average hsDNAquant at the test locus and then estimated the average hsDNA copy number by calculating the ratio to the hsDNAquant determined at each independent reference assay.

**Silhouette scores**

Silhouette scores are a graphical aid for interpretation and validation of data clusters that provides a measure of how well a data point was classified when it was assigned to a cluster (Lovmar et al. 2005). In brief, they are determined by comparing the distance between each data point within a cluster to the distance between each data point in any other cluster. The overall average silhouette score was calculated for each qicPCR assay using the software ClusterA (Lovmar et al. 2005).

**Results and Discussion**

*Evaluation of qicPCR*

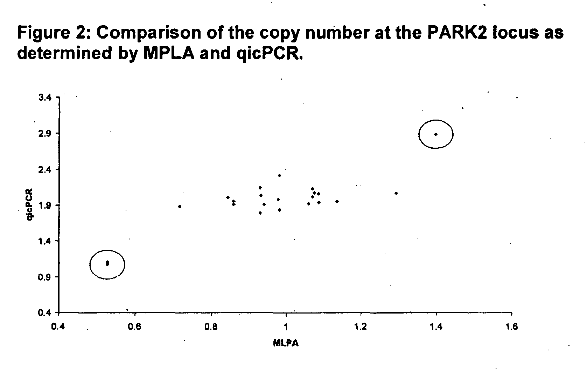

To initially evaluate qicPCR we analysed a total of 12 probes designed to assay exons at the PARK2 locus and compared the results to data generated by the established technique of MPLA (Schouten et al. 2002) . All  probes were analysed in 10 healthy controls as well as two PD individuals who had previously been characterised as carrying PARK2 mutations. Analysis of the human DNA copy number (hsCN) determined for the assays targeting PARK2 exons 3 and 6 established that qicPCR was capable of detecting both the heterozygous deletion at exon 3 (mean hsCN=1.08) and the heterozygous duplication at exon 6 (mean hsCN=2.88) in the respective PD samples (table 1) . Neither assay detected any evidence for a change in copy number in any of the 10 control samples (exon 3a; mean hsCN= 1.92 (1.8-2.0) : exon 3b; mean hsCN= 1.76 (1.66- 1.88) : exon 6; mean hsCN=2.16 (2.04=2.26) ), table 1. Direct comparison of the hsCN estimates of qicPCR to those generated by MPLA showed that the results of both methods were highly correlated, r=0.82, p=0.005 (table 1 and figure 2) . No evidence for change in copy number was observed in either the PD patients or the 10 control samples at the qicPCR assays that were not targeting PARK2 exons 3 or 6 (mean hsCN= 1.98, 95% CI; 1.9- 2.06), table 1. A total of four replicate experiments were performed for each sample. Comparison of the average hsCN determined at each technical replicate indicated that the results of qicPCR were reproducible (coefficient of variance for samples with a gain in copy, a loss in copy and also those showing no evidence of variation being 0.035, 0.09 and 0.01 respectively) . Three PARK2 exons were each assayed using two independent probes. Comparison of the hsCN determined for the 10 control samples revealed that the results for each pair of probes were consistent; exon3 (exon3a; mean hsCN= 0.96 (95% CI; 0.90- 1.01) : exon 3b; mean hsCN = 0.88 (95% CI; 0.83- 0.94) : CV = 0.06), exon 5 (exon 5a; mean hsCN = 0.93 (95% CI; 0.88- 0.98) : exon 5b; mean hsCN = 1.03 (95% CI; 0.99- 1.07) : CV = 0.07) and exon 8 (exon 8a; mean hsCN = 0.96 (95% CI; 0.91- 1.0) : exon 8b; mean hsCN = 1.01 (95% CI; 0.. 95- 1.06) : CV = 0.04), Table 1.

*Evaluation of qicPCR in large samples*

To be applicable to large scale association analysis of a given CNV, qicPCR should allow hsCN at the locus to be automatically called in >95% of samples with an error rate <0.1 %, which are the typical standards of current high throughput SNP genotyping platforms (Sawcer et al..2004). It is therefore essential that the hsCN estimates at each locus are sufficiently consistent over large numbers of samples to allow the clear distinction of those with different levels of copy number.

We used two criteria to assess the quality of the data generated by qicPCR. First, analysis of the coefficient of variance of the hsCN estimated for all samples within each category of copy number known to be present in our dataset (normal, single copy loss) allowed a specific assessment of the concordance of the data within each group. Second, we used silhouette scores (Lovmar et al. 2005) to assess the quality with which the hsDNA estimated by qicPCR formed distinct clusters. This is effectively a measure of the accuracy with which data points can be blindly assigned to different groups based on the relative location of all other data points. Silhouette scores range from- 1 to +1 with assays >0.65 generally being considered high quality, having minimal distance between the data points within each group and large distances between groups. We empirically determined the CV expected of clusters of datapoints in a high quality genotyping assay by calculating the CV for the data generated by 6 independent SNP genotyping assays (iplex, Sequenom) that we had previously scored as being high quality (>95% of samples called with an error rate <0.1 %) . All had silhouette scores >0.65 and the CV for all clusters of datapoints were <0.1 (mean= 0.05 (0.04min- 0.07max) ) . Based on this we set our criteria for genotyping quality to be silhouette score > 0.65 and CV<0.1.

To assess whether qicPCR was sufficiently robust to meet the criteria expected of a genotyping assay we analysed two independent multiplex panels each consisting of 5 independent test assays designed to detect copy number variation at chromosome 22q11 (qicPCR22q11a and qicPCR22q11b). Both multiplex assays were initially analysed in 10 individuals previously diagnosed with 22q11DS and 10 healthy controls.

In comparison to the preliminary data generated at the PARK2 locus, analysing a larger number of samples revealed that while determining the hsDNA copy number of each 22q11 test probe with comparison to a single reference probe allowed the 22q11DS samples (mean hsCN = 1.10 (0.58- 1.84) ) to be generally differentiated from the non deleted control subjects (mean hsCN = 2.14 (1.16- 3.62) ), the quality of the data fell short of that expected for a genotyping assay (mean silhouette score = 0.16 (- 0.03- 0.32) ; CV of hsCN for 22q11DS samples and non deleted controls = 0.25 and 0.28 respectively) .

We reasoned that the measurement error in the hsCN determined by qicPCR could be reduced by; 1) averaging multiple independent unlinked reference loci and/or 2) averaging multiple independent test probes targeting the same test locus. We therefore set out to systematically assess the variability associated with these parameters. Estimating hsCN using a single test probe with reference to an increasing number of independent control assays (1 to 5) resulted in tighter confidence intervals for the average estimated hsCN for both 22q11DS and non- deleted samples (figure 3a) . However, the modest reduction in the average CV (22q11DS; 0.25 to 0.19: controls 0.28 to 0.21) and a minor increase in the silhouette scores (0.16 (- 0.03min- 0.32max) to 0.32 (0.26 min- 0.36 max) ) (Figure 3a), did not achieve the required performance.

To assess the impact of increasing the number of test assays we considered all 22q11 test probes to be independent assays targeting a single CNV (the 22q11 locus) . hsDNAabsolute values were then calculated for each sample using a single 22q11 test probe and also from the average of 2, 3, 4 and 5 independent 22q11 test probes. Estimates of hsCN were then determined with reference to a single control assay amplified within the same multiplex panel. This again revealed that increasing the number of independent test assays from one to five resulted in tighter confidence intervals for the hscopy number for both 22q11DS and non- deleted samples (figure 3b), but that the minor reduction in the CV (22q11DS; 0.25 to 0.19: controls 0.28 to 0.21) and minor improvement in the silhouette scores (0.16 (- 0.03 min- 0.32 max) to 0.40 (- 0.02 min- 0.65 max) ) did not the meet the criteria expected for a genotyping assay (Figure 3b) .

We next assessed the effect of increasing both the number of independent test probes and also the number of independent control probes within the same multiplex reaction. Calculating the hsDNAquant for the locus as an average of at least 4 independent test assays and then determining the mean hsCN for the locus by comparing to at least 4 reference assays resulted in a considerable improvement in data quality (Figure 4) where the low CV<0.09 and high silhouette scores (>0.77) met our pre-defined criteria for a genotyping assay. We performed an identical assessment of qicPCR to detect changes in copy number at 22q11 using an entirely independent multiplex panel of 22q11 test assays (qicPCR22q11b). Analysis in the same set of samples revealed that the estimates of hsCN at 22q11 by each multiplex panel of markers were similar (Figure 5) and that both CV and silhouette scores met our pre defined criteria when at least 4 independent test and reference assays were used (data not presented).

Given our experience that the performance of some assays can be less robust when scaled up, we analysed a larger series of 753 samples, composed of 733 non deleted controls and twenty new 22q11DS samples independent of those in which the assay was optimised, dispersed among the control samples. For each sample the hsCN at the 22q11 locus was determined as an average of 5 locus specific test probes and 5 reference probes all located within the same multiplex reaction. All samples were analysed in duplicate and similar results were obtained from both experimental replicates (figure 6) . All 22q11DS samples could be clearly distinguished from the 20 control samples (silhouette score=0.81) (Figure 6) . The mean hsCN estimate of the 22q11DS samples was 0.96 (0.46min- 1.48max), mean CV=0.20 (0.14min- 0.23max), while that of the non deleted controls was 2.07 (1.68min- 2.82max), mean CV=0.06 (0.05min- 0.07max) . High throughput genotyping assays are expected to be sufficiently robust to analyse large numbers of samples (dropout rate <5%) while maintaining very low genotype error rates (<0.1%) . Blind genotyping of our samples using a simple Excel (Microsoft) spreadsheet which adhered to a simple semi- automated protocol whereby samples were called as carrying either 1 or 2 copy numbers if the hsCN determined by qicPCR fell within +/- 40% of the expected integer (1 copy=0.6- 1.4, 2 copies=1.6- 2.4) . Estimates of hsCN that fell outside these ranges were classed as genotype failures. Analysis of each single pass experiment resulted in >97% of samples being genotyped with 100% accuracy (a total of 46/1506 samples failed to genotype) . Moreover, determining the hsCN as the average of the two experimental replicates of each multiplex panel further improved the genotyping quality, with >99.4% of samples genotyped with 100% accuracy (a total of 5/753 samples analysed in duplicate failed to be genotyped), suggesting that further improvements in the quality of quantitative genotyping of CNVs by qicPCR are possible simply by performing one replicate experiment,

The accuracy of qicPCR is highly dependent on the reference locus itself not varying in copy number. While it is clearly impossible to absolutely eliminate rare CNVs at any given reference locus, the risk could clearly be reduced by selecting reference loci from genomic regions that have previously shown no evidence of common CNVs. Moreover, this possibility could be further reduced by designing each multiplex reaction to include multiple reference loci selected from different genomic regions, allowing rare CNVs present at just one control region to be detected and excluded from the analysis.

Another potential problem is the presence of SNPs at the primer binding sites. Most SNPs are specific to either human or chimpanzee (Kehrer-Sawatzki and Cooper 2007). Human specific SNPs (hsSNPs) located at the primer binding sites of either the test or reference locus would result in polymorphic mismatches which could lead to variability in the estimates of copy number by qicPCR due to allele specific 'drop out'. Consequently, hsSNPs located under the primers of the test and reference assays would lead to an apparent reduction and increase of the estimated copy number at the test locus respectively. Both test and reference assays should be excluded for the presence of hsSNPs, the unintentional selection which at either the target or reference locus would likely result in a highly unstable assay. Given that the qicPCR assay estimates the hscopy number with reference to only a single chimpanzee genome the presence of chimpanzee specific SNPs (ptSNPs) are likely to have minimal effect on the accuracy of a qicPCR assay, either when they have been inadvertently selected as assay targets or as when they result in mismatches under the primer sequences. However, qicPCR assays that inadvertently target ptSNPs will fail if the genotype of the reference chimpanzee DNA sample is homozygous for the same nucleotide conserved in human. Ideally ptSNPs should therefore be avoided, however as the reference chimpanzee genome sequence was primarily derived from a single donor (Clint) then this task could be aided by utilising the DNA from 'Clint' in qicPCR assays.

Single nucleotide substitutions have been estimated to occur at an average rate of 1.23% between the human and chimpanzee genome, with ~1.06% corresponding to fixed divergence between the two species (Consortium 2005). Non-conserved nucleotides are, however, not consistently distributed throughout the human genome with CpG islands, and distal regions showing increased rates of divergence (Consortium 2005). Given this, analyses of the human genome dissected into 1 Mb segments indicate that the rate of divergence with chimpanzee varies from 0.006 to 0.022 (Consortium 2005), implying that even in the most conserved regions of the human genome we could expect non-conserved nucleotides to occur on average once every ~200bp. Therefore, as the most conservative estimate suggests that non-conserved nucleotides are at least as common as SNPs it is likely that qicPCR assays could be designed to target a large majority of the human genome. Clearly, it would be impossible to analyse human sequences that do not have a chimpanzee homologue by qicPCR, but, comparative analysis of the human and chimpanzee genomic sequence indicate that this number is very small, with only 53 genes (~0.2%) being identified as being deleted in either species (Consortium 2005). The results of large scale studies aimed at genotyping CNVs by array methods can be ambiguous, and typically require validation by an independent assay. As qicPCR assays target diverged nucleotides then they can act as an independent assay to validate CNVs detected by more conventional methods (SNPs, CGH). Morever, given the high frequency of fixed non conserved nucleotides it will be potentially feasible to design qicPCR assays to analyse small CNVs (<2kb) which are becoming increasingly the target of CNV studies. In this study we have shown that qicPCR can accurately distinguish normal copy number (2 copies) from a single copy deletion (1 copy) and a single copy gain (3 copies). A number of the reports of disease association have involved more complex CNVs, typically having $\geq 4$ DNA copies (Aitman et al. 2006; Gonzalez et al. 2005; Hollox et al. 2008). While we have not analysed assays of this nature in this study, competitive PCR using the paralogue ratio test (Armour et al. 2007) has been previously demonstrated to have sufficient sensitivity to genotype complex CNVs (Armour et al. 2007; Hollox et al. 2008). Given this and the sensitivity of the data reported in this study, it is possible that qicPCR will be sufficiently sensitive to allow the genotyping of more complex copy number polymorphisms. All assays performed in this study were performed using the Sequenom Massarray genotyping platform which is best suited to analysing multiplex panels of 30-40 assays and consequently limited to analysing ~6 CNVs simultaneously. Given that qicPCR can potentially be performed using any SNP genotyping assay that generates a quantitative readout of the signal intensity for each allele, it can be applied to currently available array based platforms and therefore offers a potential that could be used in very large scale CNV genotyping studies.

**Supplementary Table 1: Primers used in qicPCR assays**

| Panel ID | ID | PCR_F | PCR_R | Extension Primer |
|---|---|---|---|---|
| qicPCR22q11a | 22q11_1 | TTACACCCGCTTTTCCAGAG | CACTGCCTACCAGAACCATC | CCAGAGGCGTTGAATC |
| | 22q11_2 | GACTCTAAAGGGCCTCCTC | CCCTAAGAGGCCTGTAGAAT | GGGGACAGCCCAGAGC |
| | 22q11_3 | TTCAAGAGCACGTCCAGCAA | CCAGTGTAGGGATCTGTTTC | tCACGTCCAGCAAGCCAGG |
| | 22q11_4 | GGTGGGAATACACAATAATA | CATGCACCTAGCAAGTAGTC | ACACAATAATATAATTTGACCAC |
| | 22q11_5 | GGATCAGATATGTTAAGCTTG | CCTGACATTTAAACATTGAC | CAGATATGTTAAGCTTGTAGTTT |
| | Reference_1 | CTCAGTGTGGTTAGAGTTGG | CTGTTCCATTTTGCAACGCC | AGAAAAGACAGATTGCAC |
| | Reference_2 | TGGAGACTTTTAGAGTCAGC | ACAAAAACGCAGCGGCTTCC | TTTTAGAGTCAGCAGGAAG |
| | Reference_3 | GTTCTAACTCTGGCTATGAC | CAAAACTGAACTCCAGAGGG | TCTGGCTATGACCTTATCTAG |
| | Reference_4 | CTCTCAATTCCCAGCTAATG | AGAGCTGGACTCTGCATTAC | agGTATCTAGGGATCCTGATGTA |
| | Reference_5 | TTTGGAAACTCACTCAGCCC | TGATTCACCAGGCAGATGTC | ccctTGAAGGAAGGTTTCGCTTTGT |
| qlcPCR22q11b | 22q11_4 | GGTGGGAATACACAATAATA | CATGCACCTAGCAAGTAGTC | ACACAATAATATAATTTGACCAC |
| | 22q11_6 | TTCTCCACTTTCTAGGTGGC | AAGAGGGTCCTGGGATCCG | cGCCCGTCCCACGGTCT |
| | 22q11_7 | AGCAAGCCATCGGCGGTGAA | TGTTCCACTGGCGGACGAT | ttcCGCACTGGACAGCGGC |
| | 22q11_8 | ATACCTGGCTCCTGCACCTT | TGTGTGACCTGGCTGCAGA | AGACCCACCTGAGGAGCTGG |
| | 22q11_9 | TGGTGCCCAGGTAACTGCAT | TGCCTCGGAGGACAAACTG | acCTGCATCCTGCCCAGCATG |
| | Reference_1 | CTCAGTGTGGTTAGAGTTGG | CTGTTCCATTTTGCAACGCC | AGAAAAGACAGATTGCAC |
| | Reference_2 | TGGAGACTTTTAGAGTCAGC | ACAAAAACGCAGCGGCTTCC | TTTTAGAGTCAGCAGGAAG |
| | Reference_3 | GTTCTAACTCTGGCTATGAC | CAAAACTGAACTCCAGAGGG | TCTGGCTATGACCTTATCTAG |
| | Reference_4 | CTCTCAATTCCCAGCTAATG | AGAGCTGGACTCTGCATTAC | agGTATCTAGGGATCCTGATGTA |
| | Reference_5 | TTTGGAAACTCACTCAGCCC | TGATTCACCAGGCAGATGTC | ccctTGAAGGAAGGTTTCGCTTTGT |
| qicPCR_PARK2a | PRKN_exon9b | AAGCAAACAAGGACAGGAAC | ACCAGAGGAAAGTCACCTGC | ACAGGAACACACCGC |
| | PRKN_exon8b | ACTGCCAGTGTTGCCTTTTG | GGGTTTGGCTTTTTTTGTGG | TTGGGAGGACACAGA |
| | PRKN_exon6a | CCAGGAAAGAGAGTTCACTG | AAAATAGCATCACTCCCCCC | CGTGTGGCAGAACAATA |
| | PRKN_exon11a | AGGCACCTTCAGACAGCATC | AGGATGTCGTGTGCTCTTTG | TCCTTTAATCCTGGAATCC |
| | PRKN_exon5b | ACATCCCTTGAAAGGGTCAC | GTGAAATTAGTGTGTAGTCC | cactGGGACCCCCAGAGTC |
| | PRKN_exon3b | GCCAGAAACAGAAATTTGGG | TCCTGCTAGATTTTATGTCC | GGGAATACGTGAAACACAA |
| | *PRKN_exon4b* | AGGCTGCTTACTTTGCTGAG | CTAACTTTATGCTCCAAGAG | gcggCACAGGGCTGTTGGC |
| | Reference_1 | CTCAGTGTGGTTAGAGTTGG | CTGTTCCATTTTGCAACGCC | AGCAGAAAAGACAGATTGCAC |

| Panel ID | ID | PCR_F | PCR_R | Extension Primer |
|---|---|---|---|---|
| qicPCR_PARK2b | PRKN_exon10a | GAATGGAACTCTCCATGACC | CTACTCATTCCTCCAAGCAG | CCAGTCCCCCACTGT |
| | PRKN_exon3a | CAAGGAGACATCACTGTAGG | CCTGTTTCATAGTACTTAC | GGTGGAGTACTTTGATGTAA |
| | PRKN_exon5a | CCTGGTCACATTCATTCCTC | GATGGAAGAACAGTCAGTTG | GACATCTTCACTGTTTGC |
| | PRKN_exon8a | GCTAATGAGACAGTTTGGGC | TCCAGCATGGTTTTCTTCCC | ggtTCTCCTGACCCTGGG |
| | Reference_1 | CTCAGTGTGGTTAGAGTTGG | CTGTTCCATTTTGCAACGCC | AGCACAAAAGACAGATTGCAC |

Supplementary Table 2: Details of Non-conserved nucleotides targeted by qicPCR

| ID | hs location (NCBI build 36.1) | | | pt location (genome build 2 version 1) | | |
|---|---|---|---|---|---|---|
| | Amplimere | non-conserved nucleotide | hs Allele | Amplimere | non-conserved nucleotide | pt Allele |
| PRKN_exon9b | chr6: 161889825-161889922 | 161889852 | T | chr6: 164535969-164536066 | 164,535,996 | C |
| PRKN_exon8b | chr6: 161910521-161910602 | 161910553 | C | chr6: 164554051-164554131 | 164,554,083 | T |
| PRKN_exon6a | chr6: 162314189-162314269 | 162314235 | T | chr6: 164967333-164967413 | 164,967,379 | C |
| PRKN_exon11a | chr6: 161701005-161701084 | 161701044 | C | chr6: 164346294-164346372 | 164,346,333 | T |
| PRKN_exon5b | chr6: 162395287-162395367 | 162395331 | A | chr6: 165047018-165047098 | 165,047,062 | G |
| PRKN_exon3b | chr6: 162603977-162604057 | 162604013 | T | chr6: 165264932-165265012 | 165,264,968 | C |
| PRKN_exon4b | chr6: 162542445-162542525 | 162542526 | A | chr6: 165198921-165198998 | 165,198,999 | G |
| PRKN_exon10a | chr6: 161727725-161727803 | 161727774 | A | Chr6: 164367262-164367340 | 164,367,311 | G |
| PRKN_exon3a | chr6: 162603341-162603417 | 162603379 | G | chr6: 165264298-165264374 | 165,264,336 | T |
| PRKN_exon5a | chr6: 162395008-162395105 | 162395049 | G | chr6: 165046739-165046836 | 165,046,780 | A |
| PRKN_exon8a | chr6: 161910241-161910327 | 161910272 | A | chr6: 164553771-164553857 | 164,553,802 | G |
| 22q11_1 | chr22: 18132605-18132704 | 18132664 | G | chr22: 18222442-18222541 | 18222500 | A |
| 22q11_2 | chr22: 18131905-18132006 | 18131961 | G | chr22: 18221742-18221843 | 18221797 | A |
| 22q11_3 | chr22: 18150388-18150500 | 78150464 | A | chr22: 18241301-18241413 | 18241377 | G |
| 22q11_4 | chr22: 18215159-18215258 | 18215215 | C | chr22: 18308804-18308903 | 18308861 | T |
| 22q11_5 | chr22: 18147129-18147232 | 18147195 | G | chr22: 18238024-18238127 | 18238090 | A |
| 22q11_6 | chr22: 18221980-18222087 | 18222015 | C | chr22: 18316460-18316567 | 18316495 | G |
| 22q11_7 | chr22: 18156280-18156390 | 18156331 | G | chr22: 18247190-18247300 | 18247240 | A |
| 22q11_8 | chr22: 18188081-18188177 | 18188133 | C | chr22: 18280511-16280607 | 18280563 | T |
| 22q11_9 | chr22: 18168814-18188918 | 18188875 | T | chr22: 18281244-18281348 | 18281304 | C |
| Reference_1 | chr2: 114545303-114545415 | 114545355 | T | chr2b: 114557982-114558094 | 114558034 | C |
| Reference_2 | chr1: 37088281-37088394 | 37088358 | A | chr1: 37399962-37400075 | 37400039 | G |
| Reference_3 | chr14: 64004080-84004177 | 64004119 | T | chr14: 63924543-63924640 | 63924582 | C |
| Reference_4 | chr5: 106790643-106790743 | 106790693 | C | chr5: 108601854-108601954 | 108601904 | T |

(continued)

| ID | hs location (NCBI build 36.1) | | | pt location (genome build 2 version 1) | | |
|---|---|---|---|---|---|---|
| | Amplimere | non-conserved nucleotide | hs Allele | Amplimere | non-conserved nucleotide | pt Allele |
| Reference 5 | chr22: 35313619-35313719 | 35313683 | C | chr22: 35461943-35462043 | 35462007 | G |

**References**

Chance, P.F., Alderson, M.K., Leppig, K.A., Lensch, M.W., Matsunami, N., Smith, B., Swanson, P.D., Odelberg, S.J., Disteche, C.M. and Bird, T.D. (1993) DNA deletion associated with hereditary neuropathy with liability to pressure palsies. Cell, 72, 143-151.

Driscoll, D.A., Spinner, N.B., Budarf, M.L., McDonald-McGinn, D.M., Zackai, E.H., Goldberg, R.B., Shprintzen, R.J., Saal, H.M., Zonana, J., Jones, M.C. et al. (1992) Deletions and microdeletions of 22q11.2 in velo-cardio-facial syndrome. Am J Med Genet, 44, 261-268.

Feuk, L., Carson, A.R. and Scherer, S.W. (2006) Structural variation in the human genome. Nat Rev Genet, 7, 85-97.

McCarroll, S.A., Hadnott, T.N., Perry, G.H., Sabeti, P.C., Zody, M.C., Barrett, J.C., Dallaire, S., Gabriel, S.B., Lee, C., Daly, M.J. et al. (2006) Common deletion polymorphisms in the human genome. Nat Genet, 38, 86-92.

Conrad, D.F., Andrews, T.D., Carter, N.P., Hurles, M.E. and Pritchard, J.K. (2006) A high-resolution survey of deletion polymorphism in the human genome. Nat Genet, 38, 75-81.

Hinds, D.A., Kloek, A.P., Jen, M., Chen, X. and Frazer, K.A. (2006) Common deletions and SNPs are in linkage disequilibrium in the human genome. Nat Genet, 38, 82-85.

Redon, R., Ishikawa, S., Fitch, K.R., Feuk, L., Perry, G.H., Andrews, T.D., Fiegler, H., Shapero, M.H., Carson, A.R., Chen, W. et al. (2006) Global variation in copy number in the human genome. Nature, 444, 444-454.

Sebat, J., Lakshmi, B., Troge, J., Alexander, J., Young, J., Lundin, P., Maner, S., Massa, H., Walker, M., Chi, M. et al. (2004) Large-scale copy number polymorphism in the human genome. Science, 305, 525-528.

Sharp, A.J., Locke, D.P., McGrath, S.D., Cheng, Z., Bailey, J.A., Vallente, R.U., Pertz, L.M., Clark, R.A., Schwartz, S., Segraves, R. et al. (2005) Segmental duplications and copy-number variation in the human genome. Am J Hum Genet, 77, 78-88.

Sebat, J., Lakshmi, B., Malhotra, D., Troge, J., Lese-Martin, C., Walsh, T., Yamrom, B., Yoon, S., Krasnitz, A., Kendall, J. et al. (2007) Strong association of de novo copy number mutations with autism. Science, 316, 445-449.

Lucito, R., Suresh, S., Walter, K., Pandey, A., Lakshmi, B., Krasnitz, A., Sebat, J., Wigler, M., Klein, A.P., Brune, K. et al. (2007) Copy-number variants in patients with a strong family history of pancreatic cancer. Cancer Biol Ther, 6, 1592-1599.

Aitman, T.J., Dong, R., Vyse, T.J., Norsworthy, P.J., Johnson, M.D., Smith, J., Mangion, J., Roberton-Lowe, C., Marshall, A.J., Petretto, E. et al. (2006) Copy number polymorphism in Fcgr3 predisposes to glomerulonephritis in rats and humans. Nature, 439, 851-855.

Gonzalez, E., Kulkarni, H., Bolivar, H., Mangano, A., Sanchez, R., Catano, G., Nibbs, R.J., Freedman, B.I., Quinones, M.P., Bamshad, M.J. et al. (2005) The influence of CCL3L1 gene-containing segmental duplications on HIV-1/AIDS susceptibility. Science, 307, 1434-1440.

Carter, N.P. (2007) Methods and strategies for analyzing copy number variation using DNA microarrays. Nat Genet, 39, S16-21.

McCarroll, S.A. and Altshuler, D.M. (2007) Copy-number variation and association studies of human disease. Nat Genet, 39, S37-42.

Moskvina, V., Craddock, N., Holmans, P., Owen, M.J. and O'Donovan, M.C. (2006) Effects of differential genotyping error rate on the type I error probability of case-control studies. Hum Hered, 61, 55-64.

Stranger, B.E., Forrest, M.S., Dunning, M., Ingle, C.E., Beazley, C., Thorne, N., Redon, R., Bird, C.P., de Grassi, A., Lee, C. et al. (2007) Relative impact of nucleotide and copy number variation on gene expression phenotypes. Science, 315, 848-853.

McCarroll, S.A. (2008) Copy-number analysis goes more than skin deep. Nat Genet, 40, 5-6.

Todd, J.A. (2006) Statistical false positive or true disease pathway? Nat Genet, 38, 731-733.

Heid, C.A., Stevens, J., Livak, K.J. and Williams, P.M. (1996) Real time quantitative PCR. Genome Res, 6, 986-994.

Charbonnier, F., Raux, G., Wang, Q., Drouot, N., Cordier, F., Limacher, J.M., Saurin, J.C., Puisieux, A., Olschwang, S. and Frebourg, T. (2000) Detection of exon deletions and duplications of the mismatch repair genes in hereditary nonpolyposis colorectal cancer families using multiplex polymerase chain reaction of short fluorescent fragments. Cancer Res, 60, 2760-2763.

Armour, J.A., Sismani, C., Patsalis, P.C. and Cross, G. (2000) Measurement of locus copy number by hybridisation with amplifiable probes. Nucleic Acids Res, 28, 605-609.

Schouten, J.P., McElgunn, C.J., Waaijer; R., Zwijnenburg, D., Diepvens, F. and Pals, G. (2002) Relative quantification of 40 nucleic acid sequences by multiplex ligation-dependent probe amplification. Nucleic Acids Res, 30, e57.

Suls, A., Claeys, K.G., Goossens, D., Harding, B., Van Luijk, R., Scheers, S., Deprez, L., Audenaert, D., Van Dyck, T., Beeckmans, S. et al. (2006) Microdeletions involving the SCN1A gene may be common in SCN1A-mutation-negative SMEI patients. Hum Mutat, 27, 914-920.

Armour, J.A., Palla, R., Zeeuwen, P.L., den Heijer, M., Schalkwijk, J. and Hollox, E.J. (2007) Accurate, high-throughput typing of copy number variation using paralogue ratios from dispersed repeats. Nucleic Acids Res, 35, e19.

Consortium, T.C.S.a.A. (2005) Initial sequence of the chimpanzee genome and comparison with the human genome. Nature, 437, 69-87.

Williams, N.M., Preece, A., Morris, D.W., Spurlock, G., Bray, N.J., Stephens, M., Norton, N., Williams, H., Clement, M., Dwyer, S. et al. (2004) Identification in 2 independent samples of a novel schizophrenia risk haplotype of the dystrobrevin binding protein gene (DTNBP1). Arch Gen Psychiatry, 61, 336-344.

Ivanov, D., Kirov, G., Norton, N., Williams, H.J., Williams, N.M., Nikolov, I., Tzwetkova, R., Stambolova, S.M., Murphy, K.C., Toncheva, D. et al. (2003) Chromosome 22q11 deletions, velo-cardio-facial syndrome and early-onset psychosis. Molecular genetic study. Br J Psychiatry, 183, 409-413.

Lovmar, L., Ahlford, A., Jonsson, M. and Syvanen, A.C. (2005) Silhouette scores for assessment of SNP genotype clusters. BMC Genomics, 6, 35.

Sawcer, S.J., Maranian, M., Singlehurst, S., Yeo, T., Compston, A., Daly, M.J., De Jager, P.L., Gabriel, S., Hafler, D.A., Ivinson, A.J. et al. (2004) Enhancing linkage analysis of complex disorders: an evaluation of high-density genotyping. Hum Mol Genet, 13, 1943-1949.

Kehrer-Sawatzki, H. and Cooper, D.N. (2007) Understanding the recent evolution of the human genome: insights from human-chimpanzee genome comparisons. Hum Mutat, 28, 99-130.

Hollox, E.J., Huffmeier, U., Zeeuwen, P.L., Palla, R., Lascorz, J., Rodijk-Olthuis, D., van de Kerkhof, P.C., Traupe, H., de Jongh, G., den Heijer, M. et al. (2008) Psoriasis is associated with increased beta-defensin genomic copy number. Nat Genet, 40, 23-25.

## Table 1: Copy number estimates at the PARK2 locus.

| ID | Test | PARK2 Assays | | | | | | | | | | |
|---|---|---|---|---|---|---|---|---|---|---|---|---|
| | | Exon 3a | Exon 3b | Exon 4b | Exon 5a | Exon 5b | Exon 6a | Exon 8a | Exon 8b | Exon 9b | Exon 10a | Exon 11a |
| PD-01 | MPLA | 1.06 | 1.06 | 2.59 | 2.17 | 2.17 | 1.88 | 2.14 | 2.14 | 2.12 | 2.15 | 2.27 |
| | qicPCR | 1.10 | 1.07 | 2.07 | 1.94 | 2.06 | 1.91 | 2.13 | 2.02 | 1.93 | 2.08 | 1.96 |
| PD-02 | MPLA | 1.96 | 1.96 | 1.95 | 1.71 | 1.71 | 2.80 | 1.86 | 1.86 | 1.68 | 1.86 | 1.43 |
| | qicPCR | 2.32 | 1.84 | 1.99 | 1.91 | 1.96 | 2.89 | 2.14 | 1.79 | 2.01 | 2.04 | 1.88 |
| Control 1 | qicPCR | 2.05 | 1.96 | 1.77 | 1.85 | 2.24 | 2.19 | 1.92 | 1.94 | 1.93 | 2.07 | 1.98 |
| Control 2 | qicPCR | 1.94 | 1.83 | 2.00 | 2.01 | 2.24 | 2.31 | 2.00 | 2.03 | 2.01 | 2.23 | 2.05 |
| Control 3 | qicPCR | 1.63 | 1.60 | 2.28 | 1.65 | 1.99 | 2.27 | 1.97 | 2.08 | 1.95 | 1.76 | 1.93 |
| Control 4 | qicPCR | 1.91 | 1.63 | 2.14 | 1.88 | 2.00 | 2.01 | 1.90 | 1.82 | 1.63 | 2.05 | 1.84 |
| Control 5 | qicPCR | 1.94 | 1.87 | 2.14 | 2.08 | 1.94 | 2.22 | 1.81 | 1.74 | 2.16 | 1.85 | 2.06 |
| Control 6 | qicPCR | 2.10 | 2.01 | 2.17 | 1.89 | 1.91 | 2.35 | 2.16 | 2.12 | 2.04 | 2.02 | 2.07 |
| Control 7 | qicPCR | 1.71 | 1.66 | 2.05 | 1.63 | 2.15 | 1.94 | 1.68 | 2.02 | 1.87 | 1.70 | 2.09 |
| Control 8 | qicPCR | 1.91 | 1.62 | 1.90 | 1.93 | 2.06 | 1.98 | 1.91 | 2.17 | 1.94 | 1.98 | 2.11 |
| Control 9 | qicPCR | 1.99 | 1.73 | 2.11 | 1.94 | 1.96 | 2.19 | 1.88 | 2.19 | 1.78 | 2.08 | 1.77 |
| Control 10 | qicPCR | 1.94 | 1.66 | 2.09 | 1.79 | 2.12 | 2.04 | 1.87 | 2.01 | 1.87 | 1.88 | 2.15 |
| | | | | | | | | | | | | |
| All Controls | Mean | 1.91 | 1.76 | 2.06 | 1.86 | 2.06 | 2.15 | 1.91 | 2.01 | 1.92 | 1.96 | 2.01 |
| | SD | 0.14 | 0.15 | 0.14 | 0.14 | 0.12 | 0.15 | 0.12 | 0.14 | 0.15 | 0.16 | 0.12 |
| | CV | 0.07 | 0.09 | 0.07 | 0.08 | 0.06 | 0.07 | 0.06 | 0.07 | 0.08 | 0.08 | 0.06 |

Organization Applicant

Street : 30-36 Newport Road
City: Cardiff
State :
Country: United Kingdom
PostalCode : CF24 0DE
PhoneNumber :
FaxNumber :
EmailAddress :

<110> OrganizationName : University College Cardiff Consultants Limited

Individual Applicant

Street: Department Psychological Medicine, School of Medicine, Cardiff University, Heath Park
City : Cardiff
State :
Country: United Kingdom
PostalCode : CF14 4XN
PhoneNumber :
FaxNumber :
EmailAddress :

<110> LastName : WILLIAMS
<110> FirstName : Nigel
<110> MiddleInitial : M
<110> Suffix : Dr

Individual Applicant

Street: Department Psychological Medicine, School of Medicine, Cardiff University, Heath-Park
City: Cardiff
State :
Country: United Kingdom
PostalCode : CF14 4XN
PhoneNumber :
FaxNumber :
EmailAddress :

<110> LastName : O'DONOVAN
<110> FirstName : Michael
<110> MiddleInitial : C
<110> Suffix : Professor

Individual Applicant

Street: Department Psychological Medicine, School of Medicine, Cardiff University, Heath Park
City: Cardiff
State :
Country: United Kingdom
PostalCode : CF14 4XN
PhoneNumber :
FaxNumber :
EmailAddress :

<110> LastName : OWEN
<110> FirstName : Michael

<110> MiddleInitial : J

< 110> Suffix : Professor

Application Project

<120> Title: Method for Determining DNA Copy Number

<130> AppFileReference: UC3.36

<140> CurrentAppNumber: GB 0811500.8

<141> CurrentFilingDate: 2008-06-20

Sequence

<213> OrganismName: Human

<400> PreSequenceString :

ttacacccgc ttttccagag          20

<212> Type: DNA

<211> Length : 20


    SequenceName : UC3.36 - SEQ ID 1

    SequenceDescription :

Sequence

<213> OrganismName : Human

<400> PreSequenceString:

cactgcctac cagaaccatc          20

<212> Type: DNA

<211> Length : 20


    SequenceName : UC3.36 - SEQ ID 2

    SequenceDescription:

Sequence

<213> OrganismName: Human

<400> PreSequenceString:

ccagaggcgt tgaatc          16

<212> Type: DNA

<211> Length : 16


    SequenceName : UC3.36 - SEQ ID 3

    SequenceDescription :

Sequence

<213> OrganismName: Human

<400> PreSequenceString:

gactctaaag ggcctcctc          19

<212> Type: DNA

<211> Length: 19


    SequenceName : UC3.36 - SEQ ID 4

    SequenceDescription :

Sequence

<213> OrganismName: Human

<400> PreSequenceString:

ccctaagagg cctgtagaat        20
<212> Type: DNA
<211> Length: 20

SequenceName : UC3.36 - SEQ ID 5
SequenceDescription:

Sequence

<213> OrganismName: Human
<400> PreSequenceString:
ggggacagcc cagagc        16
<212> Type : DNA
<211> Length : 16

SequenceName : UC3.36 - SEQ ID 6
SequenceDescription :

Sequence

<213> OrganismName: Human
<400> PreSequenceString :
ttcaagagca cgtccagcaa        20
<212> Type : DNA
<211> Length : 20

SequenceName : UC3.36- SEQ ID 7
SequenceDescription :

Sequence

<213> OrganismName : Human
<400> PreSequenceString:
ccagtgtagg gatctgtttc        20
<212> Type : DNA
<211> Length: 20

SequenceName : UC3.36 - SEQ ID 8
SequenceDescription:

Sequence

<213> OrganismName : Human
<400> PreSequenceString:
tcacgtccag caagccagg        19
<212> Type : DNA
<211> Length : 19

SequenceName: UC3.36 - SEQ ID 9
SequenceDescription :

Sequence

<213> OrganismName : Human
<400> PreSequenceString :
ggtgggaata cacaataata        20
<212> Type: DNA
<211> Length : 20

SequenceName : UC3.36 - SEQ ID 10
SequenceDescription:

Sequence

<213> OrganismName: Human
<400> PreSequenceString :
catgcaccta gcaagtagtc          20
<212> Type : DNA
<211> Length: 20

SequenceName : UC3.36 - SEQ ID 11
SequenceDescription:

Sequence

<213> OrganismName : Human
<400> PreSequenceString:
acacaataat ataatttgac cac          23
<212> Type : DNA
<211> Length : 23

SequenceName : UC3.36 - SEQ ID 12
SequenceDescription :

Sequence

<213> OrganismName: Human
<400> PreSequenceString:
ggatcagatd tgttaagctt g          21
<212> Type : DNA
<211> Length : 21

SequenceName : UC3.36 - SEQ ID 13
SequenceDescription :

Sequence

<213> OrganismName : Human
<400> PreSequenceString:
cctgacattt aaacattgac          20
<212> Type : DNA
<211> Length : 20

SequenceName: UC3.36 - SEQ ID 14
SequenceDescription:

Sequence

<213> OrganismName : Human
<400> PreSequenceString:
cagatatgtt aagcttgtag ttt          23
<212> Type : DNA
<211> Length : 23

SequenceName : UC3.36 - SEQ ID 15
SequenceDescription:

Sequence

<213> OrganismName : Human
<400> PreSequenceString:
ctcagtgtgg ttagagttgg           20
<212> Type : DNA
<211> Length : 20

    SequenceName : UC3.36 - SEQ ID 16
    SequenceDescription:

Sequence

<213> OrganismName : Human
<400> PreSequenceString :
ctgttccatt ttgcaacgcc           20
<212> Type : DNA
<211> Length : 20

    SequenceName : UC3.36 - SEQ ID 17
    SequenceDescription :

Sequence

<213> OrganismName : Human
<400> PreSequenceString :
agaaaagaca gattgcac           18
<212> Type : DNA
<211> Length: 18

    SequenceName : UC3.36 - SEQ ID 18
    SequenceDescription :

Sequence

<213> OrganismName : Human <400> PreSequenceString:
tggagacttt tagagtcagc           20
<212> Type : DNA
<211> Length: 20

    SequenceName : UC3.36 - SEQ ID 19
    SequenceDescription:

Sequence

<213> OrganismName : Human
<400> PreSequenceString:
acaaaaacgc agcggcttcc           20
<212> Type : DNA
<211> Length: 20

    SequenceName : UC3.36 - SEQ ID 20
    SequenceDescription :

Sequence

<213> OrganismName: Human
<400> PreSequenceString:

ttttagagtc agcaggaag         19
<212> Type : DNA
<211> Length : 19

SequenceName : UC3.36 - SEQ ID 21
SequenceDescription:


Sequence

<213> OrganismName : Human <400> PreSequenceString :
gttctaactc tggctatgac         20
<212> Type : DNA
<211> Length : 20

SequenceName: UC3.36 - SEQ ID 22
SequenceDescription :


Sequence

<213> OrganismName: Human
<400> PreSequenceString:
caaaactgaa ctccagaggg         20
<212> Type : DNA
<211> Length : 20

SequenceName : UC3.36 - SEQ ID 23
SequenceDescription:


Sequence

<213> OrganismName: Human
<400> PreSequenceString :
tctggctatg accttatcta g         21
<212> Type : DNA
<211> Length: 21

SequenceName : UC3.36 - SEQ ID 24
SequenceDescription:


Sequence

<213> OrganismName: Human
<400> PreSequenceString:
ctctcaattc ccagctaatg         20
<212> Type : DNA
<211> Length: 20

SequenceName: UC3.36 - SEQ ID 25
SequenceDescription :


Sequence

<213> OrganismName: Human
<400> PreSequenceString:
agagctggac tctgcattac         20
<212> Type : DNA
<211> Length: 20

SequenceName : UC3.36 - SEQ ID 26
SequenceDescription:

Sequence

<213> OrganismName: Human
<400> PreSequenceString:
aggtatctag ggatcctgat gta          23
<212> Type : DNA
<211> Length : 23

SequenceName : UC3.36 - SEQ ID 27
SequenceDescription:

Sequence

<213> OrganismName: Human
<400> PreSequenceString:
tttggaaact cactcagccc          20
<212> Type : DNA
<211> Length: 20

SequenceName : UC3.36 - SEQ ID 28
SequenceDescription:

Sequence

<213> OrganismName: Human
<400> PreSequenceString:
tgattcacca ggcagatgtc          20
<212> Type : DNA
<211> Length: 20

SequenceName : UC3.36 - SEQ ID 29
SequenceDescription:

Sequence

<213> OrganismName : Human
<400> PreSequenceString:
cccttgaagg aaggtttcgc tttgt          25
<212> Type : DNA
<211> Length: 25

SequenceName : UC3.36 - SEQ ID 30
SequenceDescription:

Sequence

<213> OrganismName: Human
<400> PreSequenceString:
ggtgggaata cacaataata          20
<212> Type : DNA
<211> Length : 20

SequenceName : UC3.36 - SEQ ID 31
SequenceDescription:

Sequence

<213> OrganismName : Human
<400> PreSequenceString :
catgcaccta gcaagtagtc          20
<212> Type : DNA
<211> Length: 20

    SequenceName : UC3.36 - SEQ ID 32
    SequenceDescription :

Sequence

<213> OrganismName : Human
<400> PreSequenceString:
acacaataat ataatttgac cac          23
<212> Type : DNA
<211> Length: 23

    SequenceName : UC3.36 - SEQ ID 33
    SequenceDescription :

Sequence

<213> OrganismName: Human
<400> PreSequenceString:
ttctccactt tctaggtggc          20
<212> Type : DNA
<211> Length: 20

    SequenceName : UC3.36 - SEQ ID 34
    SequenceDescription :

Sequence

<213> OrganismName: Human
<400> PreSequenceString:
aagagggtcc tgggatccg          19
<212> Type: DNA
<211> Length: 19

    SequenceName : UC3.36 - SEQ ID 35
    SequenceDescription :

Sequence

<213> OrganismName : Human
<400> PreSequenceString:
cgcccgtccc acggtct          17
<212> Type : DNA
<211> Length : 17

    SequenceName : UC3.36 - SEQ ID 36
    SequenceDescription:

Sequence

<213> OrganismName : Human

<400> PreSequenceString:
agcaagccat cggcggtgaa      20
<212> Type : DNA
<211> Length: 20

    SequenceName : UC3.36 - SEQ ID 37
    SequenceDescription :

Sequence

<213> OrganismName : Human
<400> PreSequenceString :
tgttccactg gcggacgat      19
<212> Type : DNA
<211> Length : 19

    SequenceName : UC3.36 - SEQ ID 38
    SequenceDescription :

Sequence

<213> OrganisimName: Human
<400> PreSequenceString :
ttccgcactg gacagcggc      19
<212> Type : DNA
<211> Length: 19

    SequenceName : UC3.36 - SEQ ID 39
    SequenceDescription:

Sequence

<213> OrganismName : Human
<400> PreSequenceString:
atacctggct cctgcacctt      20
<212> Type : DNA
<211> Length : 20

    SequenceName : UC3.36 - SEQ ID 40
    SequenceDescription :

Sequence

<213> OrganismName: Human
<400> PreSequenceString:
tgtgtgacct ggctgcaga      19
<212> Type : DNA
<211> Length : 19

    SequenceName : UC3.36 - SEQ ID 41
    SequenceDescription:

Sequence

<213> OrganismName: Human
<400> PreSequenceString:
agacccacct gaggagctgg      20
<212> Type : DNA

<211> Length: 20

SequenceName : UC3.36 - SEQ ID 42
SequenceDescription :

Sequence

<213> OrganismName : Human
<400> PreSequenceString:
tggtgcccag gtaactgcat        20
<212> Type : DNA
<21 1 > Length : 20

SequenceName : UC3.36 - SEQ ID 43
SequenceDescription:

Sequence

<213> OrganismName : Human
<400> PreSequenceString:
tgcctcggag gacaaactg        19
<212> Type : DNA
<211> Length : 19

SequenceName : UC3.36 - SEQ ID 44
SequenceDescription :

Sequence

<213> OrganismName: Human
<400> PreSequenceString:
acctgcatcc tgcccagcat g        21
<212> Type : DNA
<211> Length: 21

SequenceName: UC3.36 - SEQ ID 45
SequenceDescription :

Sequence

<213> OrganismName: Human
<400> PreSequenceString:
ctcagtgtgg ttagagttgg        20
<212> Type : DNA
<211> Length: 20 UC3.36-SEQ ID 46

SequenceName : UC3.36 - SEQ ID 46
SequenceDescription:

Sequence

<213> OrganismName: Human
<400> PreSequenceString:
ctgttccatt ttgcaacgcc        20
<212> Type : DNA
<211> Length : 20

SequenceName : UC3.36 - SEQ ID 47

SequenceDescription:

Sequence

<213> OrganismName: Human
<400> PreSequenceString:
agaaaagaca gattgcac          18
<212> Type : DNA
<211> Length: 18

SequenceName : UC3.36 - SEQ ID 48
SequenceDescription:

Sequence

<213> OrganismName: Human
<400> PreSequenceString:
tggagacttt tagagtcagc          20
<212> Type : DNA
<211> Length : 20

SequenceName : UC3.36-SEQ ID 49
SequenceDescription:

Sequence

<213> OrganismName : Human
<400> PreSequenceString:
acaaaaacgc agcggcttcc          20
<212> Type : DNA
<211> Length: 20

SequenceName: UC3.36 - SEQ ID 50
SequenceDescription:

Sequence

<213> OrganismName : Human

<400> PreSequenceString:
ttttagagtc agcaggaag          19
<212> Type : DNA
<211> Length : 19

SequenceName: UC3.36 - SEQ ID 51
SequenceDescription :

Sequence

<213> OrganismName: Human
<400> PreSequenceString:
gttctaactc tggctatgac          20
<212> Type : DNA
<211> Length: 20

SequenceName : UC3.36 - SEQ ID 52
SequenceDescription:

Sequence

<213> OrganismName : Human
<400> PreSequenceString:
caaaactgaa ctccagaggg          20
<212> Type : DNA
<211> Length: 20

    SequenceName : UC3.36 - SEQ ID 53
    SequenceDescription:

Sequence

<213> OrganismName: Human
<400> PreSequenceString:
tctggctatg accttatcta g          21
<212> Type : DNA
<211> Length : 21

    SequenceName: UC3.36 - SEQ ID 54
    SequenceDescription:

Sequence

<213> OrganismName : Human
<400> PreSequenceString:
ctctcaattc ccagctaatg          20
<212> Type : DNA
<211> Length: 20

    SequenceName : UC3.36 - SEQ ID 55
    SequenceDescription :

Sequence

<213> OrganismName: Human
<213> OrganismName: Human
<400> PreSequenceString:
agagctggac tctgcattac          20
<212> Type : DNA
<211> Length : 20

    SequenceName : UC3.36 - SEQ ID 56
    SequenceDescription :

Sequence

<213> OrganismName: Human
<400> PreSequenceString:
aggtatctag ggatcctgat gta          23
<212> Type : DNA
<211> Length : 23

    SequenceName : UC3.36 - SEQ ID 57
    SequenceDescription:

Sequence

<213> OrganismName: Human
<400> PreSequenceString:
tttggaaact cactcagccc        20
<212> Type : DNA
<211> Length : 20

SequenceName: UC3.36 - SEQ ID 58
SequenceDescription:

Sequence

<213> OrganismName: Human
<400> PreSequenceString:
tgattcacca ggcagatgtc        20
<212> Type : DNA
<211> Length: 20

SequenceName : UC3.36 - SEQ ID 59
SequenceDescription :

Sequence

<213> OrganismName: Human
<400> PreSequenceString:
cccttgaagg aaggtttcgc tttgt        25
<212> Type : DNA
<211> Length : 25

SequenceName: UC3.36 - SEQ ID 60
SequenceDescription:

Sequence

<213> OrganismName: Human
<400> PreSequenceString:
aagcaaacaa ggacaggaac        20
<212> Type : DNA
<211> Length: 20

SequenceName: UC3.36 - SEQ ID 61
SequenceDescription:

Sequence

<213> OrganismName: Human
<400> PreSequenceString:
accagaggaa agtcacctgc        20
<212> Type : DNA
<211> Length : 20

SequenceName : UC3.36 - SEQ ID 62
SequenceDescription:

Sequence

<213> OrganismName: Human
<400> PreSequenceString :
acaggaacac accgc        15

<212> Type : DNA

<211> Length: 15

SequenceName : UC3.36 - SEQ ID 63
SequenceDescription:

Sequence

<213> OrganismName : Human
<400> PreSequenceString:
actgccagtg ttgccttttg    20
<212> Type : DNA
<211> Length: 20

SequenceName UC3.36 - SEQ ID 64
SequenceDescription :

Sequence

<213> OrganismName: Human
<400> PreSequenceString :
gggtttggct ttttttgtgg    20
<212> Type : DNA
<211> Length: 20

SequenceName : UC3.36 - SEQ ID 65
SequenceDescription :

Sequence

<213> OrganismName: Human
<400> PreSequenceString:
ttgggaggac acaga    15 <212> Type : DNA

<211> Length: 15

SequenceName : UC3.36 - SEQ ID 66
SequenceDescription:

Sequence

<213> OrganismName: Human
<400> PreSequenceString:
ccaggaaaga gagttcactg    20
<212> Type : DNA
<211> Length : 20

SequenceName : UC3.36 - SEQ ID 67
SequenceDescription:

Sequence

<213> OrganismName : Human
<400> PreSequenceString:

aaaatagcat cactcccccc        20
<212> Type : DNA
<211> Length: 20

SequenceName : UC3.36 - SEQ ID 68
SequenceDescription :

Sequence

<213> OrganismName: Human
<400> PreSequenceString:
cgtgtggcag aacaata        17
<212> Type : DNA
<211> Length: 17

SequenceName : UC3.36 - SEQ ID 69
SequenceDescription:

Sequence

<213> OrganismName: Human
<400> PreSequenceString:
aggcaccttc agacagcatc        20
<212> Type : DNA
<211> Length: 20

SequenceName : UC3.36 - SEQ ID 70
SequenceDescription:

Sequence

<213> OrganismName: Human
<400> PreSequenceString:
aggatgtcgt gtgctctttg        20
<212> Type : DNA
<211> Length: 20

SequenceName : UC3.36 - SEQ ID 71
SequenceDescription:

Sequence

<213> OrganismName : Human
<400> PreSequenceString:
tcctttaatc ctggaatcc        19
<212> Type : DNA
<211> Length: 19

SequenceName : UC3.36 - SEQ ID 72
SequenceDescription:

Sequence

<213> OrganismName : Human
<400> PreSequenceString:
acatcccttg aaagggtcac        20
<212> Type : DNA
<211> Length : 20

SequenceName: UC3.36 - SEQ-ID 73
SequenceDescription:

Sequence

<213> OrganismName: Human
<400> PreSequenceString :
gtgaaattag tgtgtagtcc 20
<212> Type : DNA
<211> Length: 20

SequenceName : UC3.36 - SEQ ID 74
SequenceDescription :

Sequence

<213> OrganismName: Human
<400> PreSequenceString:
cactgggacc cccagagtc      19
<212> Type : DNA
<211> Length: 19

SequenceName : UC3.36 - SEQ ID 75
SequenceDescription :

Sequence

<213> OrganismName : Human
<400> PreSequenceString:
gccagaaaca gaaatttggg      20
<212> Type : DNA
<211> Length : 20

SequenceName : UC3.36 - SEQ ID 76
SequenceDescription :

Sequence

<213> OrganismName: Human
<400> PreSequenceString:
tcctgctaga ttttatgtcc      20
<212> Type : DNA
<211> Length : 20

SequenceName : UC3.36 - SEQ ID 77
SequenceDescription :

Sequence

<213> OrganismName : Human
<400> PreSequenceString:
gggaatacgt gaaacacaa      19
<212> Type : DNA
<211> Length: 19

SequenceName : UC3.36 - SEQ ID 78
SequenceDescription:

Sequence

<213> OrganismName: Human
<400> PreSequenceString:
aggctgctta ctttgctgag        20
<212> Type : DNA
<211> Length : 20

SequenceName : UC3.36 - SEQ ID 79
SequenceDescription:

Sequence

<213> OrganismName: Human
<400> PreSequenceString :
ctaactttat gctccaagag        20
<212> Type : DNA
<211> Length : 20

SequenceName: UC3.36 - SEQ ID 80
SequenceDescription:

Sequence

<213> OrganismName: Human
<400> PreSequenceString:
gcggcacagg gctgttggc        19
<212> Type : DNA
<211> Length : 19

SequenceName : UC3.36 - SEQ ID 81
SequenceDescription :

Sequence

<213> OrganismName: Human
<400> PreSequenceString:
ctcagtgtgg ttagagttgg        20
<212> Type: DNA
<211> Length : 20

SequenceName : UC3.36 - SEQ ID 82
SequenceDescription:

Sequence

<213> OrganismName : Human
<400> PreSequenceString:

ctgttccatt ttgcaacgcc        20
<212> Type : DNA
<211> Length: 20

SequenceName : UC3.36 - SEQ ID 83
SequenceDescription :

Sequence

<213> OrganismName : Human
<400> PreSequenceString:
agcagaaaag acagattgca c        21
<212> Type : DNA
<211> Length: 21

SequenceName : UC3.36 - SEQ ID 84
SequenceDescription:

Sequence

<213> OrganismName : Human
<400> PreSequenceString:
gaatggaact ctccatgacc        20
<212> Type : DNA
<211> Length: 20

SequenceName : UC3.36 - SEQ ID 85
SequenceDescription:

Sequence

<213> OrganismName : Human
<400> PreSequenceString:
ctactcattc ctccaagcag        20
<212> Type : DNA
<211> Length : 20

SequenceName : UC3.36 - SEQ ID 86
SequenceDescription:

Sequence

<213> OrganismName: Human
<400> PreSequenceString:
ccagtccccc actgt        15
<212> Type : DNA
<211> Length : 15

SequenceName : UC3.36 - SEQ ID 87
SequenceDescription :

Sequencer

<213> OrganismName: Human
<400> PreSequenceString:
caaggagaca tcactgtagg        20
<212> Type : DNA
<211> Length: 20

SequenceName : UC3.36 - SEQ ID 88
SequenceDescription :

Sequence

<213> OrganismName: Human
<400> PreSequenceString:
cctgtttcat agtacttac        19

<212> Type : DNA
<211> Length : 19

SequenceName : UC3.36 - SEQ ID 89
SequenceDescription:

Sequence

<213> OrganismName: Human
<400> PreSequenceString:
ggtggagtac tttgatgtaa          20
<212> Type : DNA
<211> Length : 20

SequenceName : UC3.36 - SEQ ID 90
SequenceDescription:

Sequence

<213> OrganismName : Human
<400> PreSequenceString:
cctggtcaca ttcattcctc          20
<212> Type : DNA
<211> Length : 20

SequenceName: UC3.36 - SEQ ID 91
SequenceDescription :

Sequence

<213> OrganismName : Human
<400> PreSequenceString:
gatggaagaa cagtcagttg          20
<212> Type : DNA
<211> Length : 20

SequenceName : UC3.36 - SEQ ID 92
SequenceDescription :

Sequence

<213> OrganismName : Human
<400> PreSequenceString :
gacatcttca ctgtttgc          18
<212> Type : DNA
<211> Length: 18

SequenceName UC3.36 - SEQ ID 93
SequenceDescription:

Sequence

<213> OrganismName: Human
<400> PreSequenceString:
gctaatgaga cagtttgggc          20
<212> Type : DNA
<211> Length : 20

SequenceName: UC3.36 - SEQ ID 94
SequenceDescription :

Sequence

<213> OrganismName: Human
<400> PreSequenceString:
tccagcatgg ttttcttccc            20
<212> Type : DNA
<211> Length : 20

SequenceName : UC3.36 - SEQ ID 95
SequenceDescription:

Sequence

<213> OrganismName : Human
<400> PreSequenceString :
ggttctcctg accctggg            18
<212> Type : DNA
<211> Length: 18

SequenceName : UC3.36 - SEQ ID 96
SequenceDescription:

Sequence

<213> OrganismName : Human
<400> PreSequenceString :
ctcagtgtgg ttagagttgg            20
<212> Type : DNA
<211> Length : 20

SequenceName : UC3.36 - SEQ ID 97
SequenceDescription :

Sequence

<213> OrganismName: Human
<400> PreSequenceString :
ctgttccatt ttgcaacgcc            20
<212> Type : DNA
<211> Length : 20

SequenceName : UC3.36 - SEQ ID 98
SequenceDescription:

Sequence

<213> OrganismName : Human
<400> PreSequenceString :
agcagaaaag acagattgca c            21
<212> Type : DNA
<211> Length : 21

SequenceName : UC3.36 - SEQ ID 99
SequenceDescription :

SEQUENCE LISTING

<110> University College Cardiff Consultants Limited
WILLIAMS, Nigel M
O'DONOVAN, Michael C
OWEN, Michael J

<120> Method for Determining DNA Copy Number

<130> UC3.36

<140> GB 0811500.8
<141> 2008-06-20

<160> 99

<170> PatentIn version 3.1

<210> 1
<211> 20
<212> DNA
<213> Human

<400> 1
ttacacccgc ttttccagag          20

<210> 2
<211> 20
<212> DNA
<213> Human

<400> 2
cactgcctac cagaaccatc          20

<210> 3
<211> 16
<212> DNA
<213> Human

<400> 3
ccagaggcgt tgaatc          16

<210> 4
<211> 19
<212> DNA
<213> Human

<400> 4
gactctaaag ggcctcctc          19

<210> 5
<211> 20
<212> DNA
<213> Human

<400> 5

ccctaagagg cctgtagaat        20

<210> 6
<211> 16
<212> DNA
<213> Human

<400> 6
ggggacagcc cagagc        16

<210> 7
<211> 20
<212> DNA
<213> Human

<400> 7
ttcaagagca cgtccagcaa        20

<210> 8
<211> 20
<212> DNA
<213> Human

<400> 8
ccagtgtagg gatctgtttc        20

<210> 9
<211> 19
<212> DNA
<213> Human

<400> 9
tcacgtccag caagccagg        19

<210> 10
<211> 20
<212> DNA
<213> Human

<400> 10
ggtgggaata cacaataata        20

<210> 11 1
<211> 20
<212> DNA
<213> Human

<400> 11 1
catgcaccta gcaagtagtc        20

<210> 12
<211> 23
<212> DNA
<213> Human

<400> 12
acacaataat ataatttgac cac        23

<210> 13
<211> 21
<212> DNA
<213> Human

<400> 13
ggatcagata tgttaagctt g          21

<210> 14
<211> 20
<212> DNA
<213> Human

<400> 14
cctgacattt aaacattgac          20

<210> 15
<211> 23
<212> DNA
<213> Human

<400> 15
cagatatgtt aagcttgtag ttt          23

<210> 16
<211> 20
<212> DNA
<213> Human

<400> 16
ctcagtgtgg ttagagttgg          20

<210> 17
<211> 20
<212> DNA
<213> Human

<400> 17
ctgttccatt ttgcaacgcc          20

<210> 18
<211> 18
<212> DNA
<213> Human

<400> 18
agaaaagaca gattgcac          18

<210> 19
<211> 20
<212> DNA
<213> Human

<400> 19
tggagacttt tagagtcagc          20

<210> 20
<211> 20

<212> DNA
<213> Human

<400> 20
acaaaaacgc agcggcttcc          20

<210> 21
<211> 19
<212> DNA
<213> Human

<400> 21
ttttagagtc agcaggaag           19

<210> 22
<211> 20
<212> DNA
<213> Human

<400> 22
gttctaactc tggctatgac          20

<210> 23
<211> 20
<212> DNA
<213> Human

<400> 23
caaaactgaa ctccagaggg          20

<210> 24
<211> 21
<212> DNA
<213> Human

<400> 24
tctggctatg accttatcta g        21

<210> 25
<211> 20
<212> DNA
<213> Human

<400> 25
ctctcaattc ccagctaatg          20

<210> 26
<211> 20
<212> DNA
<213> Human

<400> 26
agagctggac tctgcattac          20

<210> 27
<211> 23
<212> DNA
<213> Human

<400> 27
aggtatctag ggatcctgat gta          23

<210> 28
<211> 20
<212> DNA
<213> Human

<400> 28
tttggaaact cactcagccc          20

<210> 29
<211> 20
<212> DNA
<213> Human

<400> 29
tgattcacca ggcagatgtc          20

<210> 30
<211> 25
<212> DNA
<213> Human

<400> 30
cccttgaagg aaggtttcgc tttgt          25

<210> 31
<211> 20
<212> DNA
<213> Human

<400> 31
ggtgggaata cacaataata          20

<210> 32
<211> 20
<212> DNA
<213> Human

<400> 32
catgcaccta gcaagtagtc          20

<210> 33
<211> 23
<212> DNA
<213> Human

<400> 33
acacaataat ataatttgac cac          23

<210> 34
<211> 20
<212> DNA
<213> Human

<400> 34
ttctccactt tctaggtggc          20

<210> 35
<211> 19
<212> DNA
<213> Human

<400> 35
aagagggtcc tgggatccg           19

<210> 36
<211> 17
<212> DNA
<213> Human

<400> 36
cgcccgtccc acggtct           17

<210> 37
<211> 20
<212> DNA
<213> Human

<400> 37
agcaagccat cggcggtgaa           20

<210> 38
<211> 19
<212> DNA
<213> Human

<400> 38
tgttccactg gcggacgat           19

<210> 39
<211> 19
<212> DNA
<213> Human

<400> 39
ttccgcactg gacagcggc           19

<210> 40
<211> 20
<212> DNA
<213> Human

<400> 40
atacctggct cctgcacctt           20

<210> 41
<211> 19
<212> DNA
<213> Human

<400> 41
tgtgtgacct ggctgcaga           19

<210> 42
<211> 20

<212> DNA
<213> Human

<400> 42
agacccacct gaggagctgg     20

<210> 43
<211> 20
<212> DNA
<213> Human

<400> 43
tggtgcccag gtaactgcat     20

<210> 44
<211> 19
<212> DNA
<213> Human

<400> 44
tgcctcggag gacaaactg     19

<210> 45
<211> 21
<212> DNA
<213> Human

<400> 45
acctgcatcc tgcccagcat g     21

<210> 46
<211> 20
<212> DNA
<213> Human

<400> 46
ctcagtgtgg ttagagttgg     20

<210> 47
<211> 20
<212> DNA
<213> Human

<400> 47
ctgttccatt ttgcaacgcc     20

<210> 48
<211> 18
<212> DNA
<213> Human

<400> 48
agaaaagaca gattgcac     18

<210> 49
<211> 20
<212> DNA
<213> Human

<400> 49
tggagacttt tagagtcagc          20

<210> 50
<211> 20
<212> DNA
<213> Human

<400> 50
acaaaaacgc agcggcttcc          20

<210> 51
<211> 19
<212> DNA
<213> Human

<400> 51
ttttagagtc agcaggaag          19

<210> 52
<211> 20
<212> DNA
<213> Human

<400> 52
gttctaactc tggctatgac          20

<210> 53
<211> 20
<212> DNA
<213> Human

<400> 53
caaaactgaa ctccagaggg          20

<210> 54
<211> 21
<212> DNA
<213> Human

<400> 54
tctggctatg accttatcta g          21

<210> 55
<211> 20
<212> DNA
<213> Human

<400> 55
ctctcaattc ccagctaatg          20

<210> 56
<211> 20
<212> DNA
<213> Human

<400> 56
agagctggac tctgcattac          20

<210> 57
<211> 23
<212> DNA
<213> Human

<400> 57
aggtatctag ggatcctgat gta          23

<210> 58
<211> 20
<212> DNA
<213> Human

<400> 58
tttggaaact cactcagccc          20

<210> 59
<211> 20
<212> DNA
<213> Human

<400> 59
tgattcacca ggcagatgtc          20

<210> 60
<211> 25
<212> DNA
<213> Human

<400> 60
cccttgaagg aaggtttcgc tttgt          25

<210> 61
<211> 20
<212> DNA
<213> Human

<400> 61
aagcaaacaa ggacaggaac          20

<210> 62
<211> 20
<212> DNA
<213> Human

<400> 62
accagaggaa agtcacctgc          20

<210> 63
<211> 15
<212> DNA
<213> Human

<400> 63
acaggaacac accgc          15

<210> 64
<211> 20

<212> DNA
<213> Human

<400> 64
actgccagtg ttgccttttg          20

<210> 65
<211> 20
<212> DNA
<213> Human

<400> 65
gggtttggct ttttttgtgg          20

<210> 66
<211> 15
<212> DNA
<213> Human

<400> 66
ttgggaggac acaga               15

<210> 67
<211> 20
<212> DNA
<213> Human

<400> 67
ccaggaaaga gagttcactg          20

<210> 68
<211> 20
<212> DNA
<213> Human

<400> 68
aaaatagcat cactcccccc          20

<210> 69
<211> 17
<212> DNA
<213> Human

<400> 69
cgtgtggcag aacaata             17

<210> 70
<211> 20
<212> DNA
<213> Human

<400> 70
aggcaccttc agacagcatc          20

<210> 71
<211> 20
<212> DNA
<213> Human

<400> 71
aggatgtcgt gtgctctttg         20

<210> 72
<211> 19
<212> DNA
<213> Human

<400> 72
tcctttaatc ctggaatcc          19

<210> 73
<211> 20
<212> DNA
<213> Human

<400> 73
acatcccttg aaagggtcac         20

<210> 74
<211> 20
<212> DNA
<213> Human

<400> 74
gtgaaattag tgtgtagtcc         20

<210> 75
<211> 19
<212> DNA
<213> Human

<400> 75
cactgggacc cccagagtc          19

<210> 76
<211> 20
<212> DNA
<213> Human

<400> 76
gccagaaaca gaaatttggg         20

<210> 77
<211> 20
<212> DNA
<213> Human

<400> 77
tcctgctaga ttttatgtcc         20

<210> 78
<211> 19
<212> DNA
<213> Human

<400> 78
gggaatacgt gaaacacaa          19

<210> 79
<211> 20
<212> DNA
<213> Human

<400> 79
aggctgctta ctttgctgag          20

<210> 80
<211> 20
<212> DNA
<213> Human

<400> 80
ctaactttat gctccaagag          20

<210> 81
<211> 19
<212> DNA
<213> Human

<400> 81
gcggcacagg gctgttggc           19

<210> 82
<211> 20
<212> DNA
<213> Human

<400> 82
ctcagtgtgg ttagagttgg          20

<210> 83
<211> 20
<212> DNA
<213> Human

<400> 83
ctgttccatt ttgcaacgcc          20

<210> 84
<211> 21
<212> DNA
<213> Human

<400> 84
agcagaaaag acagattgca c        21

<210> 85
<211> 20
<212> DNA
<213> Human

<400> 85
gaatggaact ctccatgacc          20

<210> 86
<211> 20

<212> DNA
<213> Human

<400> 86
ctactcattc ctccaagcag          20

<210> 87
<211> 15
<212> DNA
<213> Human

<400> 87
ccagtccccc actgt          15

<210> 88
<211> 20
<212> DNA
<213> Human

<400> 88
caaggagaca tcactgtagg          20

<210> 89
<211> 19
<212> DNA
<213> Human

<400> 89
cctgtttcat agtacttac          19

<210> 90
<211> 20
<212> DNA
<213> Human

<400> 90
ggtggagtac tttgatgtaa          20

<210> 91
<211> 20
<212> DNA
<213> Human

<400> 91
cctggtcaca ttcattcctc          20

<210> 92
<211> 20
<212> DNA
<213> Human

<400> 92
gatggaagaa cagtcagttg          20

<210> 93
<211> 18
<212> DNA
<213> Human

<400> 93
gacatcttca ctgtttgc        18


<210> 94
<211> 20
<212> DNA
<213> Human


<400> 94
gctaatgaga cagtttgggc        20


<210> 95
<211> 20
<212> DNA
<213> Human


<400> 95
tccagcatgg ttttcttccc        20


<210> 96
<211> 18
<212> DNA
<213> Human


<400> 96
ggttctcctg accctggg        18


<210> 97
<211> 20
<212> DNA
<213> Human


<400> 97
ctcagtgtgg ttagagttgg        20


<210> 98
<211> 20
<212> DNA
<213> Human


<400> 98
ctgttccatt ttgcaacgcc        20


<210> 99
<211> 21
<212> DNA
<213> Human


<400> 99
agcagaaaag acagattgca c        21


**Claims**

1. A method for determining copy number at a specific test locus in DNA of an exploratory species, the method comprising the steps of:

   (i) co-amplifying a DNA sample of the exploratory species, i.e. the exploratory sample, comprising the test locus with at least one competitor sample test locus comprising an orthologous DNA sequence derived from a closely related species;

(ii) targeting nucleotides within the orthologous DNA sequence that are not conserved between the two species using at least one appropriate molecular assay in order to distinguish between the DNA sequences of the exploratory and competitor samples;

(iii) quantifying the amount of the DNA present in the exploratory species for each assay targeting a non-conserved nucleotide by comparing the amount of the DNA present in the exploratory species with that in the competitor sample; and

(iv) determining a quantitative estimate of the DNA copy number at the test locus in the exploratory species;

wherein the competitor sample comprises DNA which is orthologous to the DNA sequence of the exploratory species and contains at least one non-conserved nucleotide such that the exploratory and competitor samples can be distinguished by at least one appropriate molecular assay.

2. A method as claimed in claim 1, wherein the exploratory sample comprises more than one test locus.

3. A method as claimed in claim 1 or claim 2, wherein the exploratory sample comprises DNA from, or consisting of, at least one chromosome or the entire genome of the individual of the exploratory species.

4. A method as claimed in any one of claims 1 to 3, wherein the test locus or loci have been predetermined as areas of interest before the method is carried out.

5. A method as claimed in any one of claims 1 to 3, wherein the test locus or loci is previously unknown.

6. A method as claimed in any one of claims 1 to 5 wherein the test locus is a DNA sequence for which copy number variation i.e. either copy gain or copy loss, is associated with phenotypic variation in the exploratory species.

7. A method for diagnosing a disease or specific phenotype known to be associated with a change in DNA copy number of a specific chromosomal DNA sequence, i.e. the test locus, in the genome of an exploratory species, the method comprising:

(i) co-amplifying a sample of DNA of the exploratory species comprising the test locus, i.e. the exploratory sample, with at least one competitor sample test locus comprising an orthologous DNA sequence derived from a closely related species;

(ii) targeting nucleotides within the orthologous DNA sequence that are not conserved between the two species using at least one appropriate molecular assay in order to distinguish between the DNA sequences of the exploratory and competitor samples;

(iii) quantifying the amount of the DNA present in the exploratory species for each assay targeting a non-conserved nucleotide by comparing the amount of the DNA present in the exploratory species with that in the competitor sample; and

(iv) determining a quantitative estimate of the DNA copy number at the test locus in the exploratory species;

wherein the competitor sample comprises DNA which is orthologous to the DNA sequence of the exploratory sample and contains at least one non-conserved nucleotide such that the exploratory and competitor samples can be distinguished by at least one appropriate molecular assay and wherein either a gain or loss of normal DNA copy number at the test locus is associated with the disease or phenotype.

8. A method as claimed in claim 7 wherein the test locus is a DNA sequence for which copy number variation (either copy gain or copy loss) is associated with phenotypic variation in the exploratory species, for example Parkinson's disease, DiGeorge's syndrome (22q11DS), cancer, Charcot-Marie Tooth, CYP450 metabolism.

9. A method of determining an association between a copy number variant (CNV) in genomic DNA and a disease or condition, the method comprising:

(i) co-amplifying a sample of DNA from a patient of the exploratory species with the disease or condition, i.e. the exploratory sample, with at least one competitor sample comprising an orthologous DNA sequence derived from a closely related species;

(ii) targeting nucleotides within the orthologous DNA sequence that are not conserved between the two species using at least one appropriate molecular assay in order to distinguish between the DNA sequences of the exploratory and competitor samples;

(iii) quantifying the amount of the DNA present in the exploratory species for each assay targeting a non-conserved nucleotide by comparing the amount of the DNA present in the exploratory species with that in the competitor sample; and

(iv) determining a quantitative estimate of the DNA copy number at one or more test loci in the exploratory species, wherein a copy number variant at a test locus may indicate association with the disease or condition;

wherein the DNA sequence of the competitor species is orthologous to the DNA sequence of the exploratory species and contains at least one non-conserved nucleotide such that the exploratory and competitor sequences can be distinguished by at least one appropriate molecular assay.

10. A method as claimed in claim 9, wherein the test loci are not previously known.

11. A method as claimed in claim 9, wherein the test locus or loci is selected to be in an area of the genome which is suspected of being associated with the disease or condition.

12. A method as claimed in claim 11, wherein the exploratory sample comprises DNA from , or consisting of, at least one chromosome or the entire genome of the patient.

13. A method as claimed in any one of claims 9 to 12 further comprising carrying out the method using exploratory samples derived from multiple patients.

14. A method as claimed in any one of claims 9 to 13, further comprising carrying out steps (i) to (iv) using reference samples from one or more patients of the exploratory species who do not suffer from the disease or condition.

15. A method as claimed any one of claims 1 to 14, wherein the amplification step is carried out by polymerase chain reaction (PCR).

16. A method as claimed in any one of claims 1 to 15, wherein the exploratory species is *Homo sapiens.*

17. A method as claimed in claim 16, wherein the closely related species is an ape species, for example chimpanzee (*Pan troglodytes*), a gorilla species or bonobo (*Pan paniscus*).

18. A method as claimed in any one of claims 1 to 17, wherein the competitor sample comprises DNA from, or consisting of, at least one chromosome or the entire genome of said individual of the competitor species.

19. A method as claimed in any one of claims 1 to 18 wherein the non-conserved nucleotides are designed by aligning the respective orthologous genomic sequences of the exploratory and competitor species and identifying nucleotides which differ between the two species.

**Patentansprüche**

1. Verfahren zur Bestimmung der Kopienzahl an einem bestimmten DNA-Locus einer explorativen Spezies, wobei das Verfahren die folgenden Schritte umfasst:

(i) Co-Amplifizieren einer DNA-Probe einer explorativen Spezies, d.h. der explorativen Probe, die den Test-Locus enthält, mit mindestens einem Test-Locus einer Konkurrenzprobe, der eine orthologe DNA-Sequenz, die von einer nahe verwandten Spezies stammt, enthält;

(ii) Abzielen auf Nukleotide innerhalb der orthologen DNA-Sequenz, die zwischen den zwei Spezies nicht konserviert sind, unter Verwendung mindestens eines geeigneten molekularen Assays, um zwischen den DNA-Sequenzen der explorativen und der Konkurrenzprobe zu unterscheiden;

(iii) Quantifizieren der DNA-Menge, die in der explorativen Spezies vorhanden ist, bei jedem Assay, der auf ein nichtkonserviertes Nukleotid abzielt durch Vergleichen der DNA-Menge, die in der explorativen Spezies vorhanden ist mit der in der Konkurrenzprobe; und

(iv) Bestimmen einer quantitativen Schätzung der DNA-Kopienzahl am Test-Locus in der explorativen Spezies;

wobei die Konkurrenzprobe DNA umfasst, die ortholog zu der DNA-Sequenz der explorativen Spezies ist und mindestens ein nicht-konserviertes Nukleotid enthält, so dass die explorative und die Konkurrenzprobe durch mindestens einen geeigneten molekularen Assay unterschieden werden können.

2. Verfahren nach Anspruch 1, wobei die explorative Probe mehr als einen Test-Locus enthält.

3. Verfahren nach Anspruch 1 oder Anspruch 2, wobei die explorative Probe DNA von mindestens einem Chromosom oder dem gesamten Genom des Individuums der explorativen Spezies enthält oder daraus besteht.

4. Verfahren nach einem der Ansprüche 1 bis 3, wobei der Test-Locus oder die Test-Loci im Voraus als Bereiche von Interesse festgelegt wurden, bevor das Verfahren durchgeführt wird.

5. Verfahren nach einem der Ansprüche 1 bis 3, wobei der Test-Locus oder die Test-Loci vorher nicht bekannt sind.

6. Verfahren nach einem der Ansprüche 1 bis 5, wobei der Test-Locus eine DNA-Sequenz ist, für die Kopienzahlveränderung, d.h., entweder Zunahme an Kopien oder Abnahme an Kopien, mit einer phänotypischen Veränderung in der explorativen Spezies verbunden ist.

7. Verfahren zur Diagnose einer Krankheit oder eines bestimmten Phänotyps, von der bzw. dem bekannt ist, dass sie bzw. er mit einer Änderung der DNA-Kopienzahl einer bestimmten Chromosomen-DNA-Sequenz, d.h. dem Test-Locus, im Genom einer explorativen Spezies verbunden ist, wobei das Verfahren Folgendes umfasst:

(i) Co-Amplifizieren einer DNA-Probe der explorativen Spezies, die den Test-Locus enthält, d.h. der explorativen Probe, mit mindestens einem Test-Locus einer Konkurrenzprobe, der eine orthologe DNA-Sequenz, die von einer nahe verwandten Spezies stammt, enthält;
(ii) Abzielen auf Nukleotide innerhalb der orthologen DNA-Sequenz, die zwischen den zwei Spezies nicht konserviert sind, unter Verwendung mindestens eines geeigneten molekularen Assays, um zwischen den DNA-Sequenzen der explorativen und der Konkurrenzprobe zu unterscheiden;
(iii) Quantifizieren der DNA-Menge, die in der explorativen Spezies vorhanden ist, bei jedem Assay, der auf ein nichtkonserviertes Nukleotid abzielt durch Vergleichen der DNA-Menge, die in der explorativen Spezies vorhanden ist mit der in der Konkurrenzprobe; und
(iv) Bestimmen einer quantitativen Schätzung der DNA-Kopienzahl am Test-Locus in der explorativen Spezies;

wobei die Konkurrenzprobe DNA umfasst, die ortholog zu der DNA-Sequenz der explorativen Probe ist und mindestens ein nichtkonserviertes Nukleotid enthält, so dass die explorative und die Konkurrenzprobe durch mindestens einen geeigneten molekularen Assay unterschieden werden können und wobei entweder eine Zunahme oder Abnahme der normalen DNA-Kopienzahl am Test-Locus mit der Krankheit oder dem Phänotyp verbunden ist.

8. Verfahren nach Anspruch 7, wobei der Test-Locus eine DNA-Sequenz ist, für die Kopienzahlveränderung (entweder Zunahme an Kopien oder Abnahme an Kopien) mit einer phänotypischen Veränderung in der explorativen Spezies, wie beispielsweise Morbus Parkinson, DiGeorge-Syndrom (22q11DS), Krebs, Charcot-Marie-Tooth, CYP450-Stoffwechsel verbunden ist.

9. Verfahren zum Feststellen einer Verbindung zwischen einer Kopienzahlveränderung (CNV) in genomischer DNA und einer Krankheit oder einem Zustand, wobei das Verfahren Folgendes umfasst:

(i) Co-Amplifizieren einer DNA-Probe von einem Patienten der explorativen Spezies, der an der Krankheit oder dem Zustand leidet, d.h. der explorativen Probe, mit mindestens einer Konkurrenzprobe, die eine orthologe DNA-Sequenz, die von einer nahe verwandten Spezies stammt, enthält;
(ii) Abzielen auf Nukleotide innerhalb der orthologen DNA-Sequenz, die zwischen den zwei Spezies nicht konserviert sind, unter Verwendung mindestens eines geeigneten molekularen Assays, um zwischen den DNA-Sequenzen der explorativen und der Konkurrenzprobe zu unterscheiden;
(iii) Quantifizieren der DNA-Menge, die in der explorativen Spezies vorhanden ist, bei jedem Assay, der auf ein nichtkonserviertes Nukleotid abzielt durch Vergleichen der DNA-Menge, die in der explorativen Spezies vorhanden ist mit der in der Konkurrenzprobe; und
(iv) Bestimmen einer quantitativen Schätzung der DNA-Kopienzahl an einem oder mehreren Test-Loci in der explorativen Spezies, wobei eine Kopienzahlveränderung an einem Test-Lokus auf eine Verbindung mit der Krankheit oder dem Zustand hinweisen könnte;

wobei die DNA-Sequenz der Konkurrenzspezies ortholog zu der DNA-Sequenz der explorativen Spezies ist und mindestens ein nicht-konserviertes Nukleotid enthält, so dass die explorative und die Konkurrenzprobe durch mindestens einen geeigneten molekularen Assay unterschieden werden können.

**10.** Verfahren nach Anspruch 9, wobei die Test-Loci vorher nicht bekannt sind.

**11.** Verfahren nach Anspruch 9, wobei der Test-Locus oder die Test-Loci ausgewählt ist bzw. sind, so dass sie in einem Bereich des Genoms liegen, von dem vermutet wird, dass er mit der Krankheit oder dem Zustand verbunden ist.

**12.** Verfahren nach Anspruch 11, wobei die explorative Probe DNA von mindestens einem Chromosom oder dem gesamten Genom des Patienten enthält oder daraus besteht.

**13.** Verfahren nach einem der Ansprüche 9 bis 12, das ferner Durchführen des Verfahrens unter Verwendung explorativer Proben, die von mehreren Patienten stammen, umfasst.

**14.** Verfahren nach einem der Ansprüche 9 bis 13, das ferner Durchführen der Schritte (i) bis (iv) unter Verwendung von Referenzproben von einem oder mehreren Patienten der explorativen Spezies, die nicht an der Krankheit oder dem Zustand leiden, umfasst.

**15.** Verfahren nach einem beliebigen der Ansprüche 1 bis 14, wobei der Amplifizierungsschritt durch Polymerase-Kettenreaktion (PCR) durchgeführt wird.

**16.** Verfahren nach einem der Ansprüche 1 bis 15, wobei die explorative Spezies *Homo sapiens* ist.

**17.** Verfahren nach Anspruch 16, wobei die eng verwandte Spezies eine Affenspezies zum Beispiel Schimpanse (*Pan troglodytes*), eine Gorillaspezies oder Bonobo (*Pan paniscus*) ist.

**18.** Verfahren nach einem der Ansprüche 1 bis 17, wobei die Konkurrenzprobe DNA von mindestens einem Chromosom oder dem gesamten Genom des Individuums der Konkurrenzspezies enthält oder daraus besteht.

**19.** Verfahren nach einem der Ansprüche 1 bis 18, wobei die nicht-konservierten Nucleotide durch Ausrichten der jeweiligen orthologen Genomsequenzen der explorativen Spezies und der Konkurrenzspezies und Identifizieren von Nukleotiden, die zwischen den beiden Spezies verschieden sind, festgelegt werden.

**Revendications**

**1.** Procédé de détermination d'un nombre de copies au niveau d'un locus spécifique d'essai dans l'ADN d'une espèce d'exploration, ce procédé comprenant les étapes suivantes :

(i) co-amplification d'un échantillon d'ADN de l'espèce d'exploration, c.-à-d. de l'échantillon d'exploitation, comprenant le locus d'essai avec au moins un locus d'essai d'échantillon compétiteur comprenant une séquence d'ADN orthologue dérivant d'une espèce intiment liée ;
(ii) ciblage, au sein de la séquence d'ADN orthologue, des nucléotides qui ne sont pas conservés entre les deux espèces à l'aide d'au moins un test moléculaire approprié, afin de distinguer entre les séquences d'ADN des échantillons d'exploration et compétiteurs ;
(iii) quantification de la quantité d'ADN présent dans l'espèce d'exploration pour chaque test ciblant un nucléotide non conservé, par comparaison de la quantité d'ADN présent dans l'espèce d'exploration avec celle présente dans l'échantillon compétiteur ; et
(iv) détermination d'une estimation quantitative du nombre de copies d'ADN au niveau du locus d'essai dans l'espèce d'exploration ;

dans lequel l'échantillon compétiteur comprend de l'ADN qui est orthologue à la séquence d'ADN de l'espèce d'exploration et qui contient au moins un nucléotide non conservé, si bien que les échantillons d'exploration et compétiteurs peuvent être distingués selon au moins un test moléculaire approprié.

**2.** Procédé selon la revendication 1, dans lequel l'échantillon d'exploration comprend plus d'un locus d'essai.

**3.** Procédé selon la revendication 1 ou la revendication 2, dans lequel l'échantillon d'exploration comprend de l'ADN venant de ou qui est constitué de au moins un chromosome ou du génome entier de l'individu de l'espèce d'exploration.

**4.** Procédé selon l'une quelconque des revendications 1 à 3, dans lequel le locus ou les loci du test ont été prédéterminés comme des zones d'intérêt avant que le procédé ne soit mis en oeuvre.

**5.** Procédé selon l'une quelconque des revendications 1 à 3, dans lequel le locus ou les loci du test sont préalablement inconnus.

**6.** Procédé selon l'une quelconque des revendications 1 à 5, dans lequel le locus de test est une séquence d'ADN pour laquelle la variation du nombre de copies, c.-à-d. le gain de copie ou la perte de copie, est associée à la variation phénotypique dans l'espèce d'exploration.

**7.** Procédé de diagnostic d'une maladie ou d'un phénotype spécifique connu pour être associé à une modification d'un nombre de copies d'ADN d'une séquence spécifique d'ADN chromosomique, c.-à-d. le locus du test, dans le génome d'une espèce d'exploration, ce procédé comprenant les étapes suivantes :

(i) co-amplification d'un échantillon d'ADN de l'espèce d'exploration, comprenant le locus du test, c.-à-d. l'échantillon d'exploration, avec au moins un locus du test d'échantillon concurrent comprenant une séquence d'ADN orthologue dérivée d'une espèce intimement liée ;
(ii) ciblage, au sein de la séquence d'ADN orthologue, des nucléotides qui ne sont pas conservés entre les deux espèces à l'aide d'au moins un test moléculaire approprié, afin de distinguer entre les séquences d'ADN des échantillons d'exploration et compétiteurs ;
(iii) quantification de la quantité de l'ADN présent dans l'espèce d'exploration pour chaque test ciblant un nucléotide non conservé, par comparaison de la quantité d'ADN présent dans l'espèce d'exploration avec celle présente dans l'échantillon compétiteur ; et
(iv) déterminer une estimation quantitative du nombre de copies d'ADN au niveau du locus du test dans l'espèce d'exploration ;

dans lequel l'échantillon compétiteur comprend de l'ADN qui est orthologue à la séquence d'ADN de l'échantillon d'exploration et qui contient au moins un nucléotide non conservé, si bien que les échantillons d'exploration et compétiteurs peuvent être distingués selon au moins un test moléculaire approprié, et dans lequel soit un gain soit une perte du nombre normal de copies d'ADN au niveau du locus du test est associé à la maladie ou au phénotype.

**8.** Procédé selon la revendication 7, dans lequel le locus du test est une séquence d'ADN pour laquelle une variation du nombre de copies (soit un gain de copies soit une perte de copies) est associée à la variation phénotypique dans l'espèce d'exploration, par exemple la maladie de Parkinson, le syndrome de DiGeorge (22q11DS), le cancer, la maladie de Charcot-Marie Teeth, le métabolisme CYP450.

**9.** Procédé de détermination d'une association entre une variante du nombre de copies (VNC) dans l'ADN génomique et une maladie ou un état, ce procédé comprenant :

(i) la co-amplification d'un échantillon d'ADN d'un patient de l'espèce d'exploration atteinte de la maladie ou de l'état, c.-à-d. de l'échantillon d'exploration, avec au moins un échantillon compétiteur comprenant une séquence d'ADN orthologue dérivée d'une espèce intimement liée ;
(ii) le ciblage, au sein de la séquence d'ADN orthologue, des nucléotides qui ne sont pas conservés entre les deux espèces à l'aide d'au moins un test moléculaire approprié, afin de distinguer entre les séquences d'ADN des échantillons d'exploration et compétiteurs ;
(iii) la quantification de la quantité de l'ADN présent dans l'espèce d'exploration pour chaque test ciblant un nucléotide non conservé, par comparaison de la quantité d'ADN présent dans l'espèce d'exploration avec celle présente dans l'échantillon compétiteur ; et
(iv) la détermination d'une estimation quantitative du nombre de copies d'ADN au niveau du ou des loci du test dans l'espèce d'exploration, dans lequel une variante du nombre de copies au niveau d'un locus du test peut indiquer une association avec la maladie ou avec l'état ;

dans lequel la séquence d'ADN de l'espèce concurrente est orthologue à la séquence d'ADN de l'espèce d'exploration et contient au moins un nucléotide non conservé tel que les séquences d'exploration et de concurrence peuvent être distinguées selon au moins un test moléculaire approprié.

10. Procédé selon la revendication 9, dans lequel les loci du test ne sont pas connus au préalable.

11. Procédé selon la revendication 9, dans lequel le ou les loci du test sont choisis pour être dans une zone du génome dont on suspecte qu'elle est associée à la maladie ou à l'état.

12. Procédé selon la revendication 11 dans lequel l'échantillon d'exploration comprend de l'ADN provenant ou constitué d'au moins un chromosome ou le génome entier du patient.

13. Procédé selon l'une quelconque des revendications 9 à 12, comprenant en outre la mise en oeuvre du procédé utilisant des échantillons d'exploration dérivés de plusieurs patients.

14. Procédé selon l'une quelconque des revendications 9 à 13, comprenant en outre la mise en oeuvre des étapes (i) à (iv) à l'aide des échantillons de référence provenant d'un ou de plusieurs patients de l'espèce d'exploration qui ne souffrent pas de la maladie ou de l'état.

15. Procédé selon l'une quelconque des revendications 1 à 14, dans lequel l'étape d'amplification a lieu par réaction en chaîne par polymérase (PCR).

16. Procédé selon l'une quelconque des revendications 1 à 15, dans lequel l'espèce d'exploration est *Homo sapiens.*

17. Procédé selon la revendications 16, dans lequel l'espèce intimement liée est une espèce de singe, par exemple le chimpanzé (*Pan troglodytes*), une espèce de gorille ou le bonobo (*Pan paniscus*).

18. Procédé selon l'une quelconque des revendications 1 à 17, dans lequel l'échantillon compétiteur comprend de l'ADN provenant ou constitué d'au moins un chromosome ou du génome entier dudit individu de l'espèce concurrente.

19. Procédé selon l'une quelconque des revendications 1 à 18, dans lequel les nucléotides non conservés sont conçus par alignement des séquences orthologues respectives des espèces d'exploration et compétitrices et par identification de nucléotides qui diffèrent entre les deux espèces.

# Figure 1: Schematic representation of the principle of quantitative interspecies competitive PCR (qicPCR).

Figure 2: Comparison of the copy number at the PARK2 locus as determined by MPLA and qicPCR.

## Figure 3a: Effect of increasing the number of reference assays to estimate copy number by qicPCR

## Figure 3b: Effect of increasing the number of test assays to estimate copy number by qicPCR

**Figure 4: Effect of increasing both the number of reference and test assays to estimate copy number by qicPCR**

## Figure 5: Reproducibility of multiplex qicPCR assays

## Figure 6: Estimated copy number at 22q11 in 753 samples

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 0811500 A

**Non-patent literature cited in the description**

- **CHANCE, P.F. ; ALDERSON, M.K. ; LEPPIG, K.A. ; LENSCH, M.W. ; MATSUNAMI, N. ; SMITH, B. ; SWANSON, P.D. ; ODELBERG, S.J. ; DISTECHE, C.M. ; BIRD, T.D.** DNA deletion associated with hereditary neuropathy with liability to pressure palsies. *Cell,* 1993, vol. 72, 143-151
- **DRISCOLL, D.A. ; SPINNER, N.B. ; BUDARF, M.L. ; MCDONALD-MCGINN, D.M. ; ZACKAI, E.H. ; GOLDBERG, R.B. ; SHPRINTZEN, R.J. ; SAAL, H.M. ; ZONANA, J. ; JONES, M.C. et al.** Deletions and microdeletions of 22q11.2 in velo-cardio-facial syndrome. *Am J Med Genet,* 1992, vol. 44, 261-268
- **FEUK, L. ; CARSON, A.R. ; SCHERER, S.W.** Structural variation in the human genome. *Nat Rev Genet,* 2006, vol. 7, 85-97
- **MCCARROLL, S.A. ; HADNOTT, T.N. ; PERRY, G.H. ; SABETI, P.C. ; ZODY, M.C. ; BARRETT, J.C. ; DALLAIRE, S. ; GABRIEL, S.B. ; LEE, C. ; DALY, M.J. et al.** Common deletion polymorphisms in the human genome. *Nat Genet,* 2006, vol. 38, 86-92
- **CONRAD, D.F. ; ANDREWS, T.D. ; CARTER, N.P. ; HURLES, M.E. ; PRITCHARD, J.K.** A high-resolution survey of deletion polymorphism in the human genome. *Nat Genet,* 2006, vol. 38, 75-81
- **HINDS, D.A. ; KLOEK, A.P. ; JEN, M. ; CHEN, X. ; FRAZER, K.A.** Common deletions and SNPs are in linkage disequilibrium in the human genome. *Nat Genet,* 2006, vol. 38, 82-85
- **REDON, R. ; ISHIKAWA, S. ; FITCH, K.R. ; FEUK, L. ; PERRY, G.H. ; ANDREWS, T.D. ; FIEGLER, H. ; SHAPERO, M.H. ; CARSON, A.R. ; CHEN, W. et al.** Global variation in copy number in the human genome. *Nature,* 2006, vol. 444, 444-454
- **SEBAT, J. ; LAKSHMI, B. ; TROGE, J. ; ALEXANDER, J. ; YOUNG, J. ; LUNDIN, P. ; MANER, S. ; MASSA, H. ; WALKER, M. ; CHI, M. et al.** Large-scale copy number polymorphism in the human genome. *Science,* 2004, vol. 305, 525-528

- **SHARP, A.J. ; LOCKE, D.P. ; MCGRATH, S.D. ; CHENG, Z. ; BAILEY, J.A. ; VALLENTE, R.U. ; PERTZ, L.M. ; CLARK, R.A. ; SCHWARTZ, S. ; SEGRAVES, R. et al.** Segmental duplications and copy-number variation in the human genome. *Am J Hum Genet,* 2005, vol. 77, 78-88
- **SEBAT, J. ; LAKSHMI, B. ; MALHOTRA, D. ; TROGE, J. ; LESE-MARTIN, C. ; WALSH, T. ; YAMROM, B. ; YOON, S. ; KRASNITZ, A. ; KENDALL, J. et al.** Strong association of de novo copy number mutations with autism. *Science,* 2007, vol. 316, 445-449
- **LUCITO, R. ; SURESH, S. ; WALTER, K. ; PANDEY, A. ; LAKSHMI, B. ; KRASNITZ, A. ; SEBAT, J. ; WIGLER, M. ; KLEIN, A.P. ; BRUNE, K. et al.** Copy-number variants in patients with a strong family history of pancreatic cancer. *Cancer Biol Ther,* 2007, vol. 6, 1592-1599
- **AITMAN, T.J. ; DONG, R. ; VYSE, T.J. ; NORSWORTHY, P.J. ; JOHNSON, M.D. ; SMITH, J. ; MANGION, J. ; ROBERTON-LOWE, C. ; MARSHALL, A.J. ; PETRETTO, E. et al.** Copy number polymorphism in Fcgr3 predisposes to glomerulonephritis in rats and humans. *Nature,* 2006, vol. 439, 851-855
- **GONZALEZ, E. ; KULKARNI, H. ; BOLIVAR, H. ; MANGANO, A. ; SANCHEZ, R. ; CATANO, G. ; NIBBS, R.J. ; FREEDMAN, B.I. ; QUINONES, M.P. ; BAMSHAD, M.J. et al.** The influence of CCL3L1 gene-containing segmental duplications on HIV-1/AIDS susceptibility. *Science,* 2005, vol. 307, 1434-1440
- **CARTER, N.P.** Methods and strategies for analyzing copy number variation using DNA microarrays. *Nat Genet,* 2007, vol. 39, 16-21
- **MCCARROLL, S.A. ; ALTSHULER, D.M.** Copy-number variation and association studies of human disease. *Nat Genet,* 2007, vol. 39, 37-42
- **MOSKVINA, V. ; CRADDOCK, N. ; HOLMANS, P. ; OWEN, M.J. ; O'DONOVAN, M.C.** Effects of differential genotyping error rate on the type I error probability of case-control studies. *Hum Hered,* 2006, vol. 61, 55-64

- **STRANGER, B.E. ; FORREST, M.S. ; DUNNING, M. ; INGLE, C.E. ; BEAZLEY, C. ; THORNE, N. ; REDON, R. ; BIRD, C.P. ; DE GRASSI, A. ; LEE, C. et al.** Relative impact of nucleotide and copy number variation on gene expression phenotypes. *Science,* 2007, vol. 315, 848-853
- **MCCARROLL, S.A.** Copy-number analysis goes more than skin deep. *Nat Genet,* 2008, vol. 40, 5-6
- **TODD, J.A.** Statistical false positive or true disease pathway?. *Nat Genet,* 2006, vol. 38, 731-733
- **HEID, C.A. ; STEVENS, J. ; LIVAK, K.J. ; WILLIAMS, P.M.** Real time quantitative PCR. *Genome Res,* 1996, vol. 6, 986-994
- **CHARBONNIER, F. ; RAUX, G. ; WANG, Q. ; DROUOT, N. ; CORDIER, F. ; LIMACHER, J.M. ; SAURIN, J.C. ; PUISIEUX, A. ; OLSCHWANG, S. ; FREBOURG, T.** Detection of exon deletions and duplications of the mismatch repair genes in hereditary nonpolyposis colorectal cancer families using multiplex polymerase chain reaction of short fluorescent fragments. *Cancer Res,* 2000, vol. 60, 2760-2763
- **ARMOUR, J.A. ; SISMANI, C. ; PATSALIS, P.C. ; CROSS, G.** Measurement of locus copy number by hybridisation with amplifiable probes. *Nucleic Acids Res,* 2000, vol. 28, 605-609
- **SCHOUTEN, J.P. ; MCELGUNN, C.J. ; WAAIJER; R. ; ZWIJNENBURG, D. ; DIEPVENS, F. ; PALS, G.** Relative quantification of 40 nucleic acid sequences by multiplex ligation-dependent probe amplification. *Nucleic Acids Res,* 2002, vol. 30, e57
- **SULS, A. ; CLAEYS, K.G. ; GOOSSENS, D. ; HARDING, B. ; VAN LUIJK, R. ; SCHEERS, S. ; DEPREZ, L. ; AUDENAERT, D. ; VAN DYCK, T. ; BEECKMANS, S. et al.** Microdeletions involving the SCN1A gene may be common in SCN1A-mutation-negative SMEI patients. *Hum Mutat,* 2006, vol. 27, 914-920
- **ARMOUR, J.A. ; PALLA, R. ; ZEEUWEN, P.L. ; DEN HEIJER, M. ; SCHALKWIJK, J. ; HOLLOX, E.J.** Accurate, high-throughput typing of copy number variation using paralogue ratios from dispersed repeats. *Nucleic Acids Res,* 2007, vol. 35, e19
- Initial sequence of the chimpanzee genome and comparison with the human genome. *Nature,* 2005, vol. 437, 69-87
- **WILLIAMS, N.M. ; PREECE, A. ; MORRIS, D.W. ; SPURLOCK, G. ; BRAY, N.J. ; STEPHENS, M. ; NORTON, N. ; WILLIAMS, H. ; CLEMENT, M. ; DWYER, S. et al.** Identification in 2 independent samples of a novel schizophrenia risk haplotype of the dystrobrevin binding protein gene (DTNBP1. *Arch Gen Psychiatry,* 2004, vol. 61, 336-344
- **IVANOV, D. ; KIROV, G. ; NORTON, N. ; WILLIAMS, H.J. ; WILLIAMS, N.M. ; NIKOLOV, I. ; TZWETKOVA, R. ; STAMBOLOVA, S.M. ; MURPHY, K.C. ; TONCHEVA, D. et al.** Chromosome 22q11 deletions, velo-cardio-facial syndrome and early-onset psychosis. Molecular genetic study. *Br J Psychiatry,* 2003, vol. 183, 409-413
- **LOVMAR, L. ; AHLFORD, A. ; JONSSON, M. ; SYVANEN, A.C.** Silhouette scores for assessment of SNP genotype clusters. *BMC Genomics,* 2005, vol. 6, 35
- **SAWCER, S.J. ; MARANIAN, M. ; SINGLEHURST, S. ; YEO, T. ; COMPSTON, A. ; DALY, M.J. ; DE JAGER, P.L. ; GABRIEL, S. ; HAFLER, D.A. ; IVINSON, A.J. et al.** Enhancing linkage analysis of complex disorders: an evaluation of high-density genotyping. *Hum Mol Genet,* 2004, vol. 13, 1943-1949
- **KEHRER-SAWATZKI, H. ; COOPER, D.N.** Understanding the recent evolution of the human genome: insights from human-chimpanzee genome comparisons. *Hum Mutat,* 2007, vol. 28, 99-130
- **HOLLOX, E.J. ; HUFFMEIER, U. ; ZEEUWEN, P.L. ; PALLA, R. ; LASCORZ, J. ; RODIJK-OLTHUIS, D. ; VAN DE KERKHOF, P.C. ; TRAUPE, H. ; DE JONGH, G. ; DEN HEIJER, M. et al.** Psoriasis is associated with increased beta-defensin genomic copy number. *Nat Genet,* 2008, vol. 40, 23-25